# EUROPEAN PATENT APPLICATION

(11) **EP 4 834 267 A2**
(43) Date of publication of application: **04.11.2026**
(21) Application number: 26174737.2
(22) Date of filing: 27.04.2026
(51) Int. Cl.: A61B 34/20, A61B 34/30

(54) **SURGICAL TRACKING SYSTEMS AND METHODS EMPLOYING MACHINE LEARNING TO IDENTIFY TRACKING ERRORS**

(30) Priority: 28.04.2025 US 202563795825 P
(71) Applicant: Mako Surgical Corp., Weston, FL 33331 (US)
(72) Inventor: TALASAZ, Ali, Plantation, FL, 33325-2514 (US)
(74) Representative: Röthinger, Rainer

(57) **Abstract**

Tracking systems and methods are provided which employ machine learning to identify tracking errors. A tracker is coupled to a surgical object. A localizer is configured to detect a pose of the tracker and produce tracking data related to the same. Controller(s) obtain the tracking data from the localizer and input the tracking data into a machine learning model that is configured to identify an error related to the detected pose of the tracker. The controller(s) produce feedback to address the error. The machine learning model can be used for error monitoring of dynamically moving tracked surgical objects, as well as for enhancing accuracy of robot registration and/or anatomical registration.

## Description

### CROSS REFERENCE TO RELATED APPLICATIONS

The subject application claims priority to United States Provisional Patent Application No. 63/795,825, filed April 28, 2025, the entire contents of which are hereby incorporated by reference.

### BACKGROUND

In surgical settings, surgical objects are often tracked by a tracking system for several reasons. For example, surgical instruments, robotic systems, and anatomies can be tracked to provide navigated guidance of an instrument or robot relative to the anatomy. The tracking system is typically an optical tracking system, such as a camera, that detects trackers attached to the surgical objects. The tracked surgical objects often move dynamically within the operating room, thereby necessitating highly accurate tracking.

Despite the need for highly accurate tracking in the surgical setting, tracking is naturally susceptible to tracking errors. One type of tracking error relates to non-systematic or externally related factors (such as obstruction of a tracker's line of sight) that reduce detectability of the tracker. Another type of tracking error is a systematic error, wherein the tracker may be fully visible, but the detected pose of the tracker is nevertheless inaccurate. Such systematic tracking inaccuracies may occur for various reasons, such as unpredictable or non-linear motion of the tracker.

Conventional tracking systems may not accurately detect or characterize such systematic errors, particularly when the tracking error exhibits unpredictable or non-linear behavior. To the extent such errors are detected, conventional tracking systems typically compensate for the errors by halting system operation when the tracking error exceeds a default error threshold. For example, some systems halt operation if a detected velocity of the tracker exceeds a predetermined velocity. However, such mitigation schemes are one-size-fits-all approaches that fail to consider several factors, such as the type of tracker, the type or context of tracker movement, the source of the error, or the context of the surgical procedure. As a result, conventional tracking systems are incapable of evaluating whether a tracking error is acceptable given the circumstances or context in which the error occurred. Accordingly, conventional tracking systems are more susceptible to indiscriminately flagging tracking errors, thereby disrupting the surgical procedure. Moreover, conventional tracking systems are not well adapted to provide intelligent feedback to address the error because such systems do not understand the nature of the error and instead blindly rely on the default error threshold.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the tracker.

According to a second aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the tracker and predict a source of the error.

According to a third aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the tracker and predict a recommendation to address the error.

According to a fourth aspect, a surgical tracking system is provided comprising: a tracker coupled to a surgical tool; a localizer that is configured to detect a pose of the tracker and produce tracking data; and a control system coupled to the localizer and being configured to: obtain the tracking data from the localizer; input the tracking data into a machine learning model that is configured to identify an error related to the tracker and estimate an action for the surgical tool to address the error.

According to a fifth aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the tracker and predict that the error should be disregarded.

According to a sixth aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the tracker and control the tracker to address the error.

According to a seventh aspect, a surgical tracking system is provided comprising: a tracker coupled to a surgical object; a robotic manipulator comprising a robotic arm formed of links and joints; a localizer coupled to and moveable with the robotic arm and being configured to detect a pose of the tracker and produce tracking data; and a control system coupled to the localizer and being configured to: obtain the tracking data from the localizer; input the tracking data into a machine learning model that is configured to identify an error related to the tracker and control the robotic arm to adjust a pose of the localizer to address the error.

According to an eighth aspect, a surgical system is provided comprising: a tracker coupled to a surgical object; a head-mounted device comprising a display configured to be positioned in front of the eyes of a wearer of the head-mounted device; a localizer coupled to and moveable with the head-mounted device and being configured to detect a pose of the tracker and produce tracking data; and a control system coupled to the localizer and being configured to: obtain the tracking data from the localizer; input the tracking data into a machine learning model that is configured to identify an error related to the tracker and provide feedback to address the error.

According to a ninth aspect, a surgical system is provided comprising: a head-mounted device comprising a display configured to be positioned in front of the eyes of a wearer of the head-mounted device; a localizer coupled to and moveable with the head-mounted device and being configured to detect a pose of an object in an operation room (such as a human hand) and produce tracking data; and a control system coupled to the localizer and being configured to: obtain the tracking data from the localizer; input the tracking data into a machine learning model that is configured to identify an error related to the detected pose of the object and provide feedback to address the error. The object can be any surgical object, and can also be a human being, or body part such as limb(s) or hand(s). The human can be a surgeon or patient.

According to a tenth aspect, a surgical tracking system is provided comprising: a tracker coupled to a surgical object; a localizer that is configured to detect a pose of the tracker and produce tracking data; and a control system coupled to the localizer and being configured to: obtain the tracking data from the localizer; input the tracking data into a machine learning model that is configured to identify an error related to the detected pose of the tracker; and produce feedback to address the error.

According to an eleventh aspect, a surgical system is provided comprising: a tracker coupled to an anatomy; a robotic manipulator coupled to the anatomy; a localizer configured to detect a pose of the tracker and produce tracking data; and a control system coupled to the localizer and being configured to: obtain the tracking data from the localizer; input the tracking data into a machine learning model that is configured to identify an error related to the tracker and control the robotic arm to adjust a pose of the anatomy to address the error.

According to a twelfth aspect, a surgical system is provided comprising: a robotic manipulator comprising a robotic arm that is configured to support and move a surgical tool relative to an anatomy; and a surgical tracking system comprising: a manipulator tracker coupled to a component of the robotic manipulator; an anatomy tracker coupled to the anatomy; a localizer that is configured to detect a pose of the manipulator tracker and a pose of the anatomy tracker and produce tracking data related to the manipulator tracker and the anatomy tracker; and a control system coupled to the localizer and being configured to: obtain the tracking data from the localizer; input the tracking data into a machine learning model that is configured to identify an error related to the detected pose of one or both of the manipulator tracker and the anatomy tracker; and produce feedback to address the error.

According to a thirteenth aspect, a surgical system is provided comprising: a robotic manipulator comprising a base and a robotic arm coupled to the base, wherein the robotic arm supports an end effector; and a surgical tracking system comprising: a manipulator tracker coupled to the robotic manipulator; a registration tracker configured to be attached to the end effector; a localizer configured to detect a pose of the manipulator tracker and a pose of the registration tracker and produce tracking data related to the manipulator tracker and the registration tracker; and a control system coupled to the robotic manipulator and the localizer and being configured to: obtain the tracking data from the localizer; input the tracking data into a machine learning model that is configured to identify an error related to the detected pose of one or both of the manipulator tracker and the registration tracker; and produce feedback to address the error.

According to a fourteenth aspect, a surgical system is provided, comprising: a robotic manipulator comprising a robotic arm that is configured to support and move a surgical tool relative to an anatomy, wherein the surgical tool include a TCP; and a surgical tracking system comprising: a manipulator tracker coupled to a component of the robotic manipulator; an anatomy tracker coupled to the anatomy; a localizer that is configured to detect a pose of the manipulator tracker and a pose of the anatomy tracker and produce tracking data related to the manipulator tracker and the anatomy tracker; and a control system coupled to the localizer and being configured to: obtain the tracking data from the localizer; based on the tracking data, compute expected errors for the manipulator tracker and the anatomy tracker; based on the expected error, compute expected true transforms of the manipulator tracker and the anatomy tracker; based on the tracking data and the expected true transforms, compute a measured TCP value and true TCP value with respect to a coordinate system of the anatomy; compute an error between the measured and true TCP values; and determine whether the error exceeds a threshold.

A computer-implemented method is provided for performing any of the steps implemented by the control system of any one or more of the preceding aspects. A non-transitory computer readable medium or computer program product is provided, comprising instructions, which when executed by one or more processors, are configured to implement any feature of the one or more of the preceding aspects.

Any of the aspects described above can be combined in part, or in whole. Any of the above aspects can be utilized in part, or whole, with any of the following implementations:

The localizer can implement optical, electromagnetic, inertial, radio frequency or machine-vision based tracking. The tracker can include tracking elements that are detectable by the localizer. The tracker can be coupled to any surgical object, such as a surgical instrument; an anatomy; a head-mounted display device; a link of a robotic arm of robotic manipulator; an adjustable arm coupled to a base of a robotic manipulator; an end effector coupled to a robotic manipulator; and/or a coupler located between a robotic arm of a manipulator and an end effector supported by the robotic arm. The tracker can include a manipulator tracker, an anatomy tracker, a tool tracker, or a registration tracker used during a registration process.

The control system can acquire identity data for the tracker. The control system can input the identity data into the machine learning model to enable the machine learning model to identify the error. The identity data can include a geometry of the tracking elements of the tracker. The tracking data can include the position and/or orientation of the tracker. The tracking data can include a velocity and/or an acceleration of the tracker. The tracking data can further include velocities of a manipulator tracker and an anatomy tracker. The control system can further utilize a tracker type associated with each tracker to compute expected errors.

The machine learning model includes a (artificial) neural network, such as a deep learning neural network. The neural network comprises an input layer, hidden layers, and an output layer, wherein the input layer is configured to receive the tracking data, and the output layer is configured to identify the error. The machine learning model can include at least one long short-term memory (LSTM) coupled to the input layer of the neural network, wherein the at least one LSTM comprises a recurrent neural network (RNN) and is configured to receive the tracking data over time, modify weights of the RNN to learn long-term dependencies among the tracking data and to selectively filter the tracking data, and provide filtered tracking data to the input layer of the neural network. The machine learning model can compute expected errors for one or more trackers based any one or more of the tracking data, identity data, and tracker type.

The error can relate to inaccuracy of the detected pose of the tracker. The machine learning model can be pre-trained based on ground truth data derived from laser tracking of the tracker or other trackers. The machine learning model can identify the error based on a comparison between the tracking data and the ground truth data. The machine learning model can establish an error threshold or range that dynamically changes based on the tracking data. The machine learning model can identify the error in response to the error exceeding the error threshold or range. The machine learning model can predict a source of the error. The source can be any one or more of: undesired movement of the surgical object; undesired movement of the localizer; or improper calibration data of the localizer or tracker. The machine learning model can estimate an impact that the error has on accuracy of one or more of: the localizer, the robotic manipulator, or the anatomy. The error can include a difference between measured and true tool center point (TCP) values, and the control system can compute the error using a registration transform of the manipulator derived from a robot registration process and a kinematic transform of the manipulator.

The control system can provide feedback to address the error. The control system can provide feedback with a notification or warning to inform a user of the error. The control system can provide feedback by selectively adjusting or replacing the tracking data to offset the error. The control system can provide feedback by filtering the tracking data. The control system can provide feedback by controlling the tracker. The control system can provide feedback by adjusting the operation or positioning of the localizer. The control system can provide feedback during a robot registration process or an anatomical registration process by filtering tracking data using the machine learning model, comparing filtered and unfiltered tracking data to kinematic data or a virtual model, presenting multiple registration results on a display, and prompting a user to select one of the results. The control system can further provide a recommendation to correct the error, and can input force data from a surgical probe into the machine learning model to identify an error associated with interaction with unwanted tissue.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of a surgical system, according to one implementation.
Figure 2 is a block diagram of an example control system for controlling the surgical system.
Figure 3 is a functional block diagram for using a machine learning model to identify tracking errors and for producing feedback to address the tracking error, according to one implementation.
Figure 4A illustrates an example of dynamically monitoring the relationship between a robotic manipulator, an anatomy, and a localizer to identify tracking errors, according to one example.
Figure 4B is a functional block diagram for using the machine learning model to identify tracking errors during dynamic error monitoring, such as shown in FIG. 4A.
Figure 4C is a functional block diagram of another implementation for identifying tracking errors during dynamic error monitoring.
Figures 5A-5D illustrate various types of manipulator trackers that can be involved with a robot registration process.
Figure 6 is a functional block diagram for using the machine learning model to identify tracking errors during robot registration, according to one implementation.
Figures 7A-7C illustrate an anatomical registration process involving a tracked anatomical region, a virtual model of the anatomy, a tracked pointer.
Figure 8 is a functional block diagram for using the machine learning model to identify tracking errors during anatomical registration, according to one implementation.

### DETAILED DESCRIPTION

### I. Example System Overview

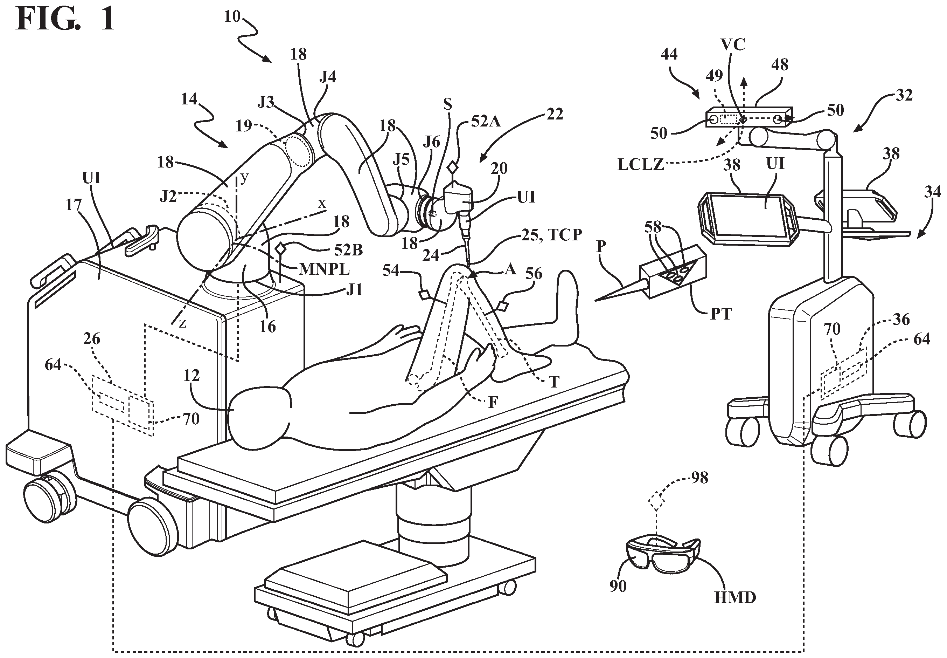

Referring to Figure 1, a surgical/medical system 10 is illustrated. As will be described below, the system 10 can be used for surgical/medical purposes in collaboration with a surgeon or patient, respectively.

For surgery, the system 10 is useful for treating a surgical site or target anatomy (A) of a patient 12, such as treating bone or soft tissue. In Figure 1, the patient 12 is undergoing a surgical procedure. The anatomy (A) in Figure 1 includes a femur F and a tibia T of the patient 12. The surgical procedure may involve tissue removal or other forms of treatment. Treatment may include cutting, coagulating, lesioning the tissue, other in-situ tissue treatments, or the like. In some examples, the surgical procedure involves partial or total knee or hip replacement surgery, shoulder replacement surgery, spine surgery, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled, "Prosthetic Implant and Method of Implantation," the disclosure of which is hereby incorporated by reference. The system 10 and techniques disclosed herein may be utilized to perform other procedures, surgical or nonsurgical, or may be utilized in industrial applications or other applications where robotic systems are utilized.

The surgical/medical system 10 may include a manipulator 14 or robotic manipulator. The manipulator 14 can be a collaborative surgical robot. Collaborative means that the surgeon/patient directly interacts with the manipulator 14 by applying forces to the manipulator 14. The manipulator 14 has a base 16 and plurality of links 18 and joints (J). A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in Figure 1), a parallel arm configuration, or any other suitable manipulator configuration. In other examples, more than one manipulator 14 may be utilized in a multiple arm configuration. In other configurations, the manipulator 14 can include a partially or entirely adjustable arm (AA), with passive joints that are manually adjustable. The joints of the adjustable arm (AA) optionally may be locked in position by the user. In other instances, some or all the joints of the adjustable arm (AA) may be actively driven using joint motors.

The manipulator 14 can include a passive (manually articulated) joint, such as planarly extending joint. For instance, the passive joint may be the distal most joint attached to the robotic arm comprising a plurality of active joints. The planarly extending joint can support a tool such as a saw blade to allow the user to manually move the saw blade along a plane. In this case, the tool is mechanically constrained to the plane by the mechanical constraints of the passive joint, while the pose of the plane is actively constrained by the active joints (J) of the robotic arm. In other instances, one or more joints (J) of the manipulator 14 could be controlled to constrain the tool in certain degrees of freedom, while allowing the tool free passive motion in other degrees of freedom or manners, such as a rotation about a point or a selected linear motion.

The manipulator 14 can exhibit kinematic redundancy wherein the tool positions are defined by up to 6DOF, but the robotic arm may operate in more than 6DOF. With such redundancy, the manipulator 14 can utilize null-space controls whereby the joints (J) of the manipulator 14 can change positions while maintaining the pose of the end effector 22. Null-space controls can also be used to avoid singularities.

In the example shown in Figure 1, the manipulator 14 comprises a plurality of joints (J) and a plurality of joint encoders 19 located at the joints (J) for determining position data of the joints (J). For simplicity, only one joint encoder 19 is illustrated in Figure 1, although other joint encoders 19 may be similarly illustrated. The manipulator 14, according to one example, has six joints J1-J6 implementing at least six-degrees of freedom (DOF) for the manipulator 14. However, the manipulator 14 may have any number of degrees of freedom and may have any suitable number of joints J and may have redundant joints. The manipulator 14 need not require joint encoders 19 but may alternatively, or additionally, utilize motor encoders present on motors at each joint J. Also, the manipulator 14 need not require rotary joints, but may alternatively, or additionally, utilize one or more prismatic joints. Any suitable combination of joint types is contemplated.

The base 16 of the manipulator 14 is a portion of the manipulator 14 that provides a fixed reference coordinate system for other components of the manipulator 14 or the system 10 in general. The origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17.

In other examples, the manipulator 14 can be a hand-held manipulator where the base 16 is a base portion of a tool (e.g., a portion held free hand by the user), and the tool tip is movable relative to the base portion. The base portion has a reference coordinate system that is tracked, and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined. The hand-held manipulator 14 can be like that described and shown in US20230255701, entitled "Systems and Methods for Guiding Movement of a Handheld Medical Robotic Instrument", the entire disclosure of which is hereby incorporated by reference.

The manipulator 14 and/or manipulator cart 17 house a manipulator controller 26, or other type of control unit. The manipulator controller 26 may comprise one or more computers, or any other suitable form of controller that directs the motion of the manipulator 14. The manipulator controller 26 may have a central processing unit (CPU), graphics processing unit (GPU) and/or other processors, memory, and storage. The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

A tool 20 couples to the manipulator 14 and is movable relative to the base 16 to interact with the anatomy (A) in certain modes. The tool 20 is a physical and surgical tool and is, or forms part of, the end effector 22 supported by the manipulator 14 in certain implementations. The tool 20 or end effector 22 may be grasped by the user. One possible arrangement of the manipulator 14 and the tool 20 is described in U.S. Patent No. 9,119,655, entitled, "Surgical Manipulator Capable of Controlling a Surgical Instrument in Multiple Modes," the disclosure of which is hereby incorporated by reference. The manipulator 14 and the end effector 22 may be arranged in alternative configurations. The end effector 22 can be like that shown in U.S. Patent Application Publication No. 2014/0276949, filed on March 15, 2014, entitled, "End Effector of a Surgical Robotic Manipulator," hereby incorporated by reference.

The tool 20 can include an energy applicator 24 designed to contact and remove the tissue of the patient 12 at the surgical site. In one example, the energy applicator 24 is a bur 25. The bur 25 may be substantially spherical and comprise a spherical center, radius (r), and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form. In other examples, the tool 20 does not include an energy applicator 24. For example, the tool 20 can be a slotted cut guide for a saw, a guide tube or tool guide for receiving another tool, or the like.

The end effector 22 may comprise a tool controller to control operation of the end effector 22, such as to control power to the tool (e.g., to a rotary motor of the tool 20), control movement of the tool 20, control irrigation/aspiration of the tool 20, and/or the like. The tool controller may be in communication with the manipulator controller 26 or other components. The manipulator controller 26 controls a state (position and/or orientation) of the tool 20 (e.g., the TCP) with respect to a coordinate system, such as the manipulator coordinate system MNPL. The manipulator controller 26 can control (linear or angular) velocity, acceleration, or other derivatives of motion of the tool 20. The tool center point (TCP), in one example, is a predetermined reference point defined at the energy applicator 24. The TCP has a known, or able to be calculated (i.e., not necessarily static), pose relative to other coordinate systems. The geometry of the energy applicator 24 is known in or defined relative to a TCP coordinate system. The TCP may be located at the spherical center of the bur 25 of the tool 20 such that only one point is tracked. The TCP may be defined in several ways depending on the configuration of the energy applicator 24. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 20 is not limited to a center point. For example, any suitable primitives, meshes, etc., can be utilized to represent the tool 20.

The manipulator 14 can operate in a variety of modes. In a free mode, the manipulator moves the end effector 22 in response to user applied forces on the manipulator 14. In a manual mode, the manipulator moves the end effector 22 in response to user applied forces but constraints the end effector 22 relative to haptic objects or virtual boundaries/constraints. In the guided-manual mode, the manipulator moves the end effector 22 in response to user applied forces but constraints the end effector 22 to a predetermined tool path. In a semi-automated or automated mode, the manipulator automatically moves the end effector 22 along a predetermined tool path, and optionally, pursuant to supervisory control by the surgeon.

The surgical/medical system 10 may include a tracking system 32 or navigation system. The tracking/navigation system 32 tracks movement of various surgical objects. Such objects include, for example, the manipulator 14, the tool 20/end effector 22 and the anatomy (A), e.g., femur F and tibia T, or patient limbs, or any other surgical tool/instrument/device. The tracking/navigation system 32 tracks these surgical objects to gather state information of each object with respect to a (navigation) localizer coordinate system LCLZ. Coordinates in the localizer coordinate system LCLZ may be transformed to the manipulator coordinate system MNPL, and/or vice-versa, using transformations. One example of the tracking/navigation system 32 is described in U.S. Patent No. 9,008,757, filed on September 24, 2013, entitled, "Navigation System Including Optical and Non-Optical Sensors," hereby incorporated by reference.

The tracking/navigation system 32 includes a cart assembly 34 that houses a navigation controller 36, and/or other types of control units. A navigation user interface UI is in operative communication with the navigation controller 36. The navigation user interface includes one or more displays 38. The tracking/navigation system 32 can display a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of: push buttons, a keyboard, a mouse, a microphone (voice-activation), gesture control devices, and the like.

The tracking/navigation system 32 also includes a localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The tracking/navigation system 32 includes one or more trackers. In one example, the trackers include a pointer tracker PT, one or more manipulator trackers 52A, 52B, a first patient tracker 54, and a second patient tracker 56. In the illustrated example of Figure 1, the manipulator tracker is rigidly attached to the end effector 22 (i.e., tracker 52A), the first patient tracker 54 is coupled to the femur F of the patient 12, and the second patient tracker 56 is coupled to the tibia T of the patient 12. The patient trackers 54, 56 may be firmly affixed to sections of bone or could be coupled to the exterior of the leg. The pointer tracker PT is firmly affixed to a pointer or probe P utilized for registering the anatomy (A) to the localizer coordinate system LCLZ. The pointer P may be hand-held or may optionally be coupled to the adjustable arm (AA), as shown in FIG. 1. The pointer P may include a force/torque sensor that is configured to measure forces/torques applied to the pointer tip. The pointer P may also include a controller, e.g., housed within the pointer body to process measurements from the force/torque sensor. Measurements from the pointer P may be communicated to the control system 60 using a wired or wireless connection.

The manipulator tracker 52A, 52B may be affixed to any suitable component of the manipulator 14, in addition to, or other than the end effector 22, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object with which it is associated.

Any one or more of the trackers may include active tracking elements 58. The active tracking elements 58 may include light emitting diodes (LEDs). Alternatively, the trackers 52A, 52B, 54, 56, PT may have passive tracking elements, such as reflectors, which reflect light emitted from the camera unit 46. Other suitable tracking elements not specifically described herein may be utilized.

The localizer 44 tracks the trackers 52A, 52B, 54, 56, PT to determine the state of each of the trackers 52A, 52B, 54, 56, PT, which correspond respectively to the state of the object respectively attached thereto. The localizer 44 may perform known triangulation techniques to determine the states of the trackers 52, 54, 56, PT, and associated objects. The localizer 44 provides the state of the trackers 52A, 52B, 54, 56, PT to the navigation controller 36. In one example, the navigation controller 36 determines and communicates the state the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. Navigation controller 36 may have a central processing unit (CPU), graphics processing unit (GPU) and/or other processors, non-transitory memory, and storage. The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that can conduct the functions described herein. The term processor is not intended to limit any embodiment to a single processor.

The tracking/navigation system 32 may have any other suitable configuration for tracking surgical objects. For instance, the tracking/navigation system 32 and/or localizer 44 can be radio frequency (RF)-based. For example, the tracking/navigation system 32 may comprise an RF transceiver coupled to the navigation controller 36. The tracker may comprise RF tracking elements (e.g., emitters or transponders) attached thereto. The RF tracking elements may be passive or actively energized. The RF transceiver transmits an RF tracking signal and generates state signals to the navigation controller 36 based on RF signals received from the RF tracking elements. The navigation controller 36 may analyze the received RF signals to associate relative states thereto. The RF signals may be of any suitable frequency. The RF transceiver may be positioned at any suitable location to track the objects using RF signals effectively. Furthermore, the RF tracking elements may have any suitable structural configuration that may be much different than the trackers 52A, 52B, 54, 56, PT shown in Figure 1.

In another example, the tracker can be detected using inertial sensing, such as using accelerometers, gyroscopes, magnetometers, MEMS sensors, or the like. The tracking/navigation system 32 can retrieve the inertial sensor information from the tracker using any suitable communication scheme, such as IR, RF, EM communication, or the like.

In yet another example, the tracking/navigation system 32 and/or localizer 44 are electromagnetically based. For example, the tracking/navigation system 32 may comprise an EM transceiver coupled to the navigation controller 36. The tracker may comprise EM tracking elements attached thereto, such as any suitable magnetic tracker, electro-magnetic tracker, inductive tracker, or the like. The EM tracking elements may be passive or actively energized. The EM transceiver generates an EM field and generates state signals to the navigation controller 36 based upon EM signals received from the EM tracking elements. The navigation controller 36 may analyze the received EM signals to associate relative states thereto. Again, such navigation system examples may have structural configurations that are different than the tracking/navigation system 32 configuration shown in Figure 1.

Alternatively, the tracking/navigation system 32 and/or localizer 44 utilize computer/machine vision. Here, the localizer 44 comprises a vision camera to identify the anatomy (A) of the patient and/or any other surgical object in the operating room using computer/machine vision technology. This technique may be utilized with a tracker that includes an optically detectable tracking elements (such as a QR code, bar code, or fiducial array). Alternatively, this technique may be trackerless. The computer/machine vision system utilizes the camera to detect movement of these objects by continuously taking images, reviewing the images, and detecting movement of the objects. The computer/machine vision system can also identify and characterize surgical objects. The computer/machine vision system may utilize any suitable image processing algorithm for determining such distance information, including, but not limited to utilizing a depth map generation, segmentation, edge detection, color analysis, blob detection, pixel analysis, pattern recognition, machine learning models, and the like. These algorithms can be executed by any one or more of the controllers described herein.

In another example, the tracking/navigation system 32 and/or localizer 44 are ultrasound-based. For example, the tracking/navigation system 32 may comprise an ultrasound imaging device coupled to the navigation controller 36. The ultrasound imaging device images any surgical object (or tracker) and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in Figure 1.

The tracking/navigation system 32 may have any other suitable components or structure not specifically recited herein. Furthermore, any of the techniques, methods, and/or components described above with respect to the tracking/navigation system 32 shown may be implemented or provided for any of the other examples of the tracking/navigation system 32 described herein. The localizer 44 can be supported by the cart assembly 34 (as shown) or can be located on any other device/support, including being attached to and moveable with the manipulator 14 or attached to a moveable/adjustable/robotic arm other than the manipulator 14.

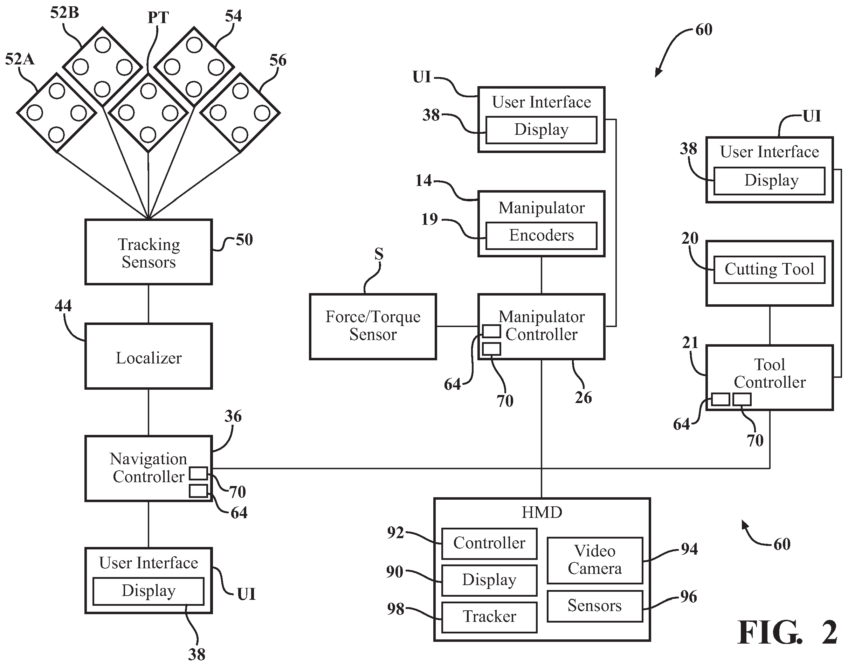

Referring to Figure 2, the system 10 includes the control system 60 that comprises, among other components, the manipulator controller 26, the navigation controller 36, and the tool controller 21. The control system 60 further includes one or more software programs and software modules. The software modules may be part of the program or programs that operate on the manipulator controller 26, navigation controller 36, tool controller 21, or any combination thereof, to process data to assist with control of the system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database.

The control system 60 may comprise any suitable configuration of input, output, and processing devices suitable for conducting the functions and methods described herein. The control system 60 may comprise the manipulator controller 26, the navigation controller 36, or the tool controller 21, or any combination thereof, or may comprise only one of these controllers. These controllers may communicate via a wired bus or communication network as shown in Figure 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that can perform the functions described herein.

The surgical system 10 may also employ a head-mounted device (HMD), as shown in FIGS. 1 and 2. The HMD includes a display 90, an HMD controller 92, a video camera 94, sensors 96, and optionally, a tracker 98. The display 90 is positioned in front of the eyes of the wearer. The display 90 may be see-through transparent or may be a digital display. The HMD can present computer-generated imagery combined with, or overlaid onto, realworld views in an extended (virtual/augmented/mixed) reality manner. The video camera 94 can detect surgical objects in the operation room and hence can function as one example of the localizer 44. The sensors 96 can detect HMD movement or can detect gestures of the wearer to provide input commands. The tracker 98 can be attached to the HMD and can be detected by localizer 44. The tracker 98 can be for any tracking modality, such as computer vision or optical tracking. The HMD controller 92 can communicate and control any of the described components of the HMD.

### II. Techniques for Employing Machine Learning Models to Identify Tracking Errors

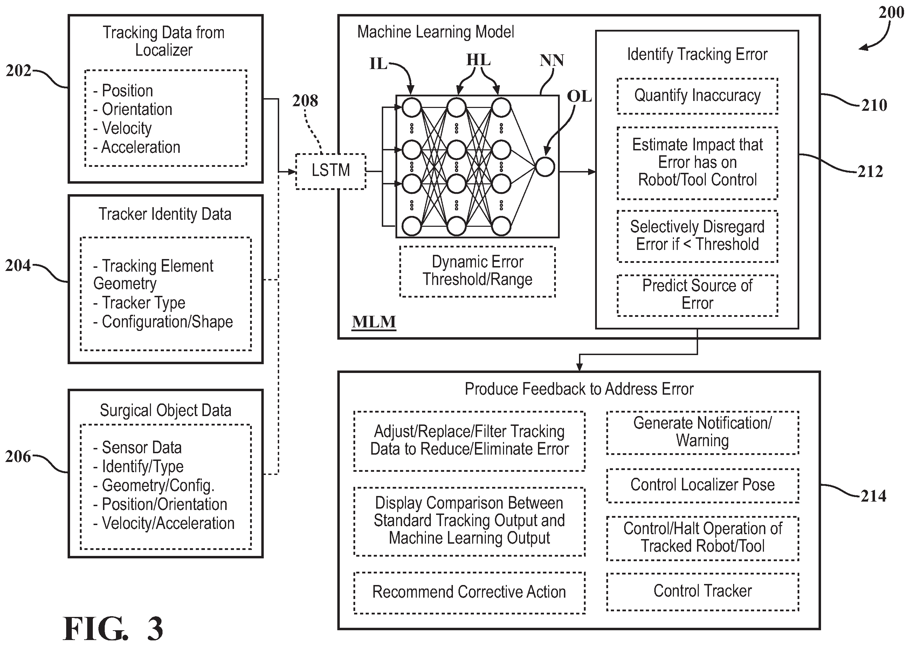

Described herein, and with continued reference to FIGS. 3-8, are systems, computer-implemented methods, computer program products, and techniques for utilizing machine learning model(s) to identify tracking errors of the tracking/navigation system 32. The techniques described herein, including the machine learning model (MLM), can be implemented by any one or more of the described controllers (e.g., navigation controller 36, manipulation controller 26, tool controller 21, HMD controller 92, etc.), which will hereinafter be described as "controller(s)" or "control system" 60.

The tracking errors can be identified with respect to any one or more of the described trackers (52, 54, 56, 98), such as those coupled to any of the described surgical objects, including but not limited to: the pointer P, a component of the manipulator 14, the HMD, the anatomy (A), or any other surgical tool/instrument. Regarding the manipulator 14, the tracker can be coupled to any one or more of the links 18 of a robotic arm of robotic manipulator 14; an adjustable arm coupled to the base 16 of a robotic manipulator 14; the end effector 22 coupled to a robotic manipulator 14; and/or a coupler located between a robotic arm of a manipulator 14 and the end effector 22 supported by the robotic arm. Example of manipulator trackers can be like those described in US Patent App. No. 18/198,938, entitled "Robotic System With Improved Configurations For Base Tracker"; example link trackers can be like those described in US Patent App. Pub. No. 20230277256, entitled, "Robotic System Including A Link Tracker"; and example trackers located on a coupler can be like those described in US Patent App. Pub. No. 20220039898, entitled, "Robotic Surgical System Including A Coupler For Connecting A Tool To A Manipulator And Methods Of Using The Coupler", the entire contents of each of these references being hereby incorporated by reference.

The techniques described herein can identify tracking errors for diverse types of trackers used in robot tracking, dynamic bone tracking, and bone registration during robotics-assisted arthroplasty. These trackers can be of various shapes and sizes and can be tracked using any of the modalities described (e.g., RF, EM, inertial, machine/computer vision, ultrasound, or any combination thereof, etc.)

The tracking errors can be related to inaccuracy of the detected pose of any one or more of the described trackers. In other words, the pose of the tracker is detected, but the detected pose is inaccurate. For example, the tracking error can be due to positional and/or orientational poses or movements of the tracker that produce sub-optimal tracking accuracy. In particular, orientational movement of the tracker tends to introduce non-linearities by causing the face of the tracker to move away from the direct line of sight of the localizer 44. In turn, such orientational movements can produce inaccuracies in optical tracking systems. The tracking error can also be identified when the tracker is partially detected or when the tracker fails to be detected. In other examples, tracking errors may result from various other causes, such as partial line-of-sight obstructions, improper calibration data or tracker operation, unexpected bumping or movement of a tracker, the localizer 44, the manipulator 14, or the like.

### A. Tracking Error Identification and Compensation

With reference to FIG. 3, described is a block diagram of a method 200 of tracking error identification and compensation that can be performed by the controller(s) using a machine learning model (MLM). The machine learning model (MLM) can advantageously identify complex and non-linear systematic tracking errors in the localization system which cannot otherwise be captured using conventional techniques. Such benefits are compounded by utilizing supplemental data, such as tracker identity and surgical object data, which will be described below. When tracker identity data is provided as an input, the machine learning model (MLM) can make higher order predictions related to the tracker error, such as errors that are unique to the specific tracker being detected and can generalize this learned experience to identify errors related to other tracker types that have not been processed before. When surgical object is provided as an input, the machine learning model (MLM) can determine complex relationships between the tracker error and the surgical environment or surgical context in a manner not previously attainable. The machine learning model (MLM) can quantify the error, estimate the impact of the error, selectively disregard the error based on a dynamic error threshold/gauge/range, and even predict the source of the error. Several feedback schemes are also contemplated for addressing or compensating for the error.

The method 200 includes, at step 202, the controller(s) obtaining tracking data from the localizer 44. In one example, the tracking data is pursuant to the localizer 44 detecting a pose of the tracker. The tracking data may include the pose (position and/or orientation) of the tracker, the velocity of the tracker, and/or the acceleration of the tracker. The tracking data may be acquired discretely or continuously over any specified duration. In some cases, the tracking data may be based on other circumstances, such as when the localizer 44 partially/incompletely, or fails to detect a pose of the tracker. The described method 200 can be utilized for any example or use case described herein, such as for robot registration, anatomical registration, dynamic error monitoring, or for tracking any one or more trackers in the surgical workspace for any other reason.

Optionally, at step 204, the controller(s) can obtain tracker identity data. The tracker identity data relates to the specific tracker(s) being detected by the localizer 44. The tracker identity data specifies information that helps identify the tracker. In one example, the tracker identity data includes information about the tracking elements of the tracker. As described, the localizer can implement various tracking modalities (e.g., optical, electromagnetic, inertial, radio frequency or computer/machine-vision based tracking). The tracker includes tracking elements that are detectable by the localizer 44 for the given tracking modality. The tracker identity data can include the tracking modality, configuration, or geometry of the tracking elements. For instance, the geometry of the tracking elements can define how the tracking elements are geometrically arranged relative to one another and relative to the body of the tracker (or the surgical object to which the tracker is coupled). Tracking element configurations can specify features such as the transmission wavelength/frequency, transmission power, intensity, or other operating parameters of the tracking elements. Tracker identity data can also include a serial number for the specific tracker or a geometrical shape of the tracker body. The tracker identity data can be acquired by the localizer 44 through initialization of the tracker or by tracking the tracker. In other cases, tracker identity data can be acquired from other sources, such as the navigation system software or memory, or in response to the user inputting the type of tracker into the software.

In some cases, at 206, the controller(s) can further obtain surgical object data. This data specifies any information that can be used to identify the surgical object being tracked by the tracker. The surgical object data can include an identification or type of surgical object being tracked. For example, the identity can specify that the surgical object being tracked is a distal femur, link (L2) of the manipulator 14, or the pointer P tool, etc. The surgical object data can include sensor data. For example, if the surgical object is a tool/robot that includes a sensor, the sensor measurements can be included with the surgical object data. Such sensors can include any sensor appropriate for the surgical object. For instance, when the pointer P is equipped with a force sensor, the sensor data may include force measurements. When the surgical object being tracked is the manipulator 14, the sensor data can include kinematic data from encoders, force/torque measurements from a force/torque sensor attached to the robotic arm, etc. Additionally, surgical object data can include pose/velocity/acceleration data related to the surgical object, which can be acquired from the sensor data, the localizer 44, or any other source (such as an external sensing system).

At 208, the tracking data (as well as the tracker identity data and surgical object data) can optionally be inputted into at least one long-short term memory (LSTM). The LSTM may be part of the machine learning model (MLM) or may be separate therefrom. There may be one LSTM for all input data, or there may be numerous LSTMs for distinct types or subsets of data. The one or more LSTM's may be implemented using a recurrent neural network (RNN) and configured to receive the input data over time. The LSTM modifies the weights of the RNN to learn long-term dependencies among the input data and to selectively filter the input data. The LSTM then provides filtered input data to a neural network (NN) of the machine learning model (MLM).

The neural network (NN) of the machine learning model (MLM) is an artificial neural network that comprises an input layer (IL), hidden layers (HL), and an output layer (OL). The input layer (IL) is configured to receive the input data (i.e., tracking data, and optionally, identity data and surgical object data). The output layer (OL) is configured to identify the tracking error.

The neural network (NN) can be any suitable type of neural network architecture, including but not limited to: deep-learning neural network, a deep-reinforcement learning neural network, a recurrent neural network (RNN), a multilayer perceptron (MLP), a feed-forward neural network, generative adversarial networks (GAN), a convolutional neural network (CNN), or the like.

The input layer (IL) nodes may receive the input data directly from the respective data sources (202, 204, 206) or may receive the filtered input data from the one or more LSTMs. The input layer (IL) may have any suitable number of nodes. One input node can receive all input data, or a node can be provided for each type of input data. In another example, there may be an input node for various subsets of each data type. For instance, for tracking data, there may be one input node for position data, another input node for velocity data, and another input node for acceleration data.

The hidden layers (HL) are networked between the input and output layers and are configured to transform the input data by modifying weights and biases and implementing activation functions. The hidden layers (HL) enable deep learning to learn complex relationships among the input data. The hidden layers (HL) transform the input data into a data representation suitable for the output layer. The neural network (NN) can include any number of hidden layers (HL).

At the output layer (OL) of the neural network (NN), the machine learning model (MLM) can make identifications, predictions, classifications, or characterizations regarding the tracking error (shown at 212). The machine learning model (MLM) can produce various results depending on its configuration or training.

In one example, the machine learning model (MLM) can quantify the tracking inaccuracy. The machine learning model (MLM) can produce a delta (difference) between the tracking data and ground truth data. The machine learning model (MLM) can be pre-trained based on ground truth data derived from laser tracking of the tracker or other trackers. Such training data may be derived from a training process wherein an array of spherically mounted reflectors (SMRs), which are pre-calibrated precision optical components, are attached to a robotic arm or other apparatus that can be manually moved. A laser tracker system then emits laser beams to the SMR's each time the SMRs to are automatically/manually moved to various poses. The SMR reflects the laser beam back to the laser tracker system and measures the distances/angles at each specific SMR pose to establish the ground truth data. Simultaneously, a version of the localizer 44 is located in the workspace and measures the positions of the measuring elements (e.g., fiducials in the case of optical systems), which have already been calibrated with respect to the SMR poses using a CMM or similar method. The tracking data from the localizer 44 is then compared to the laser tracking data to establish a difference between the tracking data and the ground truth data for several poses in the localizer's workspace. The machine learning model (MLM) can characterize the delta between the tracking data and ground truth data using any suitable format, such as with delta with respect to each of the six-degrees of freedom (i.e., 3 DOF position and 3DOF orientation) of the tracker pose.

In another example, the machine learning model (MLM) can estimate an impact that the tracking error has on the surgical object to which the tracker is attached. For Example, the machine learning model (MLM) can estimate the impact on control of the manipulator 14 or other tool attached to the tracker to provide dynamic error monitoring which can provide a risk-assessment scheme, e.g., during robotic resection procedures, Examples of such dynamic error monitoring will be described below.

The machine learning model (MLM) can also intelligently and selectively evaluate whether the tracking error should be disregarded by establishing an error threshold/range/gauge that dynamically changes based on the input data (tracking data, identity data, surgical object data) and/or identified error. The error threshold/range/gauge can change for each instance of tracking data. The machine learning model (MLM) can identify the error in response to the error exceeding the dynamically changing error threshold/range/gauge. In other cases, the error threshold/range/gauge could be a user set preference. This error threshold/range/gauge can be adjusted during the procedure. There could be different error thresholds/ranges/gauges based on the intended accuracy for the step of the surgical procedure. The threshold selection can be context-dependent and adjusted based on procedural phases and the intended accuracy. In some instances, the error threshold/range/gauge may be automatically customized based on a maximum expected error for a specific surgical step or task, such as for performing a specific resection cut. Cutting away large areas of bone may have a high threshold with more acceptable errors. A precise cut near the boundaries of the plan may have a lower threshold for acceptable error. The machine learning model (MLM) may dynamically change the error threshold/range/gauge based upon a distance from a critical boundary, for example. In another example, the expected cutting error at TCP can be mapped into one or more active constraint zones and reactive constraint zones (e.g., for a TKA planar cut). In the active constraint zones, the system 10 should follow the surgical plan closely by keeping the tool precisely on the specific cutting plane. Because of the need for high accuracy in active constraint zone, the machine learning model (MLM) can set a low and fixed error limit to prevent inter-cut errors. For the error mapped into the reactive constraint zones, the machine learning model (MLM) can set a higher threshold since it does not affect accuracy until a boundary is penetrated. At that point, the threshold can be set low to avoid un-intended penetration. The error threshold/range/gauge can be changed according to any suitable mathematical function, such as a linear (e.g., spring constant) or non-linear function. Examples of active and reactive constraint schemes that can be utilized with the techniques described herein can be like those described in U.S. Provisional Patent App. No. 63/737,587, filed December 20, 2024, entitled "Systems And Methods Employing Machine Learning Models To Improve Surgical Robotic Performance", the entire contents of which are hereby incorporated by reference.

The machine learning model (MLM) can also predict a source of the error. The source can be undesired movement of the surgical object or undesired movement of the localizer 44. Undesired movement may be due to the localizer 44 or surgical object being bumped or moved unexpectedly. A primary source of error may be a change in the orientation of the tracker or movement of the localizer 44, which in turn may cause unintended orientation changes of the tracker(s) used in the application. The orientation change of the tracker may be a normal or unexpected orientational change, both of which can affect tracking accuracy. Any orientation error introduced at the localizer can have an amplified effect on the TCP error when propagated along the kinematic chain. Specifically, rotational errors can induce additional translational errors at the TCP due to the lever-arm effect, where small angular deviations cause larger positional shifts over distance. The magnitude of this translational error depends on the tracker's location within the kinematic chain: the farther the tracker is from both the TCP and the anatomy tracker, the more significant the impact on positional accuracy. Consequently, inaccuracies in tracker orientation can directly translate into increased cutting errors, particularly when the tracker is mounted at a point remote from the resection area. In other examples, the source of the error may be improper calibration data of the localizer 44 or tracker. In other examples, the source of the error may be a line-of-sight obstruction between the localizer 44 and the tracker. Such obstructions may be from an object (such as human) being located between the localizer 44 and the tracker or may result from debris being located on an optical tracking element of the tracker. The source may also be a malfunctioning of a component or feature of the localizer 44 or tracker. For instance, one of the tracking elements may not be emitting signals, or the tracker battery may be low thereby providing insufficient power to LED tracking elements. In another example, the source of the error may be that the tracker is out of range or out of the field-of-view of the localizer 44. By learning complex relationships among input data (tracking data, identity data, surgical object data), the machine learning model (MLM) can make accurate predictions about the source of the error thereby eliminating guess work or troubleshooting in the operating room, which can consume valuable time.

At 214, the controller(s) can produce feedback to address the tracking error. This feedback can be provided in numerous manners depending on the circumstances.

In one example, the controller(s) and/or the machine learning model (MLM) can provide feedback by selectively adjusting, replacing, or filtering the tracking data to offset or eliminate the error. Such compensation can include adjusting, replacing, or filtering any of the six-degrees of freedom values of the tracking data that identifies tracker pose. Such compensation schemes may be useful during robot or anatomical registration processes, which will be described below. Such selective adjusting, replacing, or filtering of the tracking data can be done near-instantaneously (e.g., on the fly- during tracking) or can be done in batches or at discrete times (e.g., after tracking, or after robot or anatomical registration processes are completed).

In another example, feedback can include a notification or warning to inform a user of the error. The warning or notification can be provided in any suitable manner, including audible, visual, haptic, or a combination thereof. In one example, the notification or warning is provided on the user interface UI or display 38 of the tracking system 32 or as a computer-generated extended reality element on the display 90 of the HMD. In another example, the notification or warning is provided on the surgical object being tracked. For example, a surgical tool or the manipulator 14 being tracked and operated by the user may vibrate or provide an audible/visual warning. Additionally, the tracker itself may provide the warning, for example, through an error indicator light or display screen on the tracker. The notification or warning can inform the user of the error as well as more advanced information determined by the machine learning model (MLM). For instance, the predicted source of the error or recommendations of corrective actions to address the source of the error can be presented. The notification may display "Warning - anatomy tracker error detected from leg movement: Stabilize femur using leg holder." If the error is selectively disregarded, the user can also be informed of such to provide transparency in the tracking data integrity. The level of inaccuracy of the error could also be provided to the user.

Other information that can be displayed can include a comparison between original tracking data and machine learning modified tracking data. For example, if the machine learning model (MLM) selectively adjusts, replaces, or filters the tracking data, the user interface UI or display 38, 90 can provide an output that allows the user to understand the difference between original tracking data and machine-learning filtered tracking data. Examples of such outputs will be described below in relation to robot or anatomical registration processes.

The controller(s) and/or the machine learning model (MLM) can also provide feedback by effectuating some sort of control of the surgical object being tracked. For example, if the manipulator 14 or surgical tool is being tracked, their operational functions may be constrained, reduced, or halted in response to the identified error. If the surgical tool is a drill, tap, or screwdriver, the tool may be inhibited from further rotational actuation in response to the error. Likewise, the manipulator 14 may be controlled in various manners in response to the identified error, such as by: adjusting commanded poses of the TCP, deviating from a tool path, following a new tool path, changing tool path direction, reducing/stopping its feed rate (tool path velocity), reducing/stopping the cutting speed of the end effector 22 (e.g., saw, cutting burr, etc.), implementing a new or modified haptic constraint or virtual boundary on the end effector 22, pulling the end effector 22 away from the surgical site (to a safe location), etc.

In another example, the tracker itself can be controlled to address the error. For example, the tracker or tracking element operation can be modified by changing signal intensity, frequency, channel, power, modulation, firing order, tracking modality, etc. If the tracker is attached to a moveable device, such as the manipulator 14 or actively adjustable arm, the manipulator 14 or adjustable arm may be controlled to change its pose to correspondingly change the pose of the tracker to address the error.

In other implementations, the localizer 44 can be controlled to address the error. For instance, the pose of the localizer 44 can be adjusted. If the localizer 44 is attached to a moveable device, such as the manipulator 14 or actively adjustable arm, the manipulator 14 or adjustable arm may be controlled to change its pose to correspondingly change the pose of the localizer 44 to address the error. Additionally, the sensing elements of the localizer 44 can be changed to address the error. For example, the sensing elements of the localizer 44 can increase exposure times or can dynamically "zoom in" on a region of interest of its field of view that encompasses the tracker. The localizer 44 can also selectively increase the amount of sensing elements that are used to compensate for the error as well as increase tracking frequency or modifying image compression.

Additionally, the anatomy (A) can be manipulated to address the error. For instance, the anatomy (A) can be adjusted pursuant to guidance provided on the display(s) 38, 90. The guidance can indicate the current location of the anatomy (A) based on the tracking data as well as the desired location of the anatomy (A). The display(s) 38, 90 may present graphics or templates to assist the user with moving the anatomy (A) into the desired position. One example system that can provide such guidance can be like that described in U.S. Patent No. 11,166,781, entitled "System, Method And Software Program For Aiding In Positioning Of A Camera Relative To A Tracker In A Surgical Environment", the entire contents of which are hereby incorporated by reference. In another example, a robotic manipulator can be attached to the anatomy (A). The localizer 44 can detect a pose of the tracker 56 attached to the anatomy (A) and produce tracking data. The controller(s) input the tracking data into the machine learning model (MLM) that is configured to identify an error related to the tracker 56. Based on this error, the controller(s) can control the robotic arm to adjust a pose of the anatomy (A) to address the error.

### B. Dynamic Error Monitoring

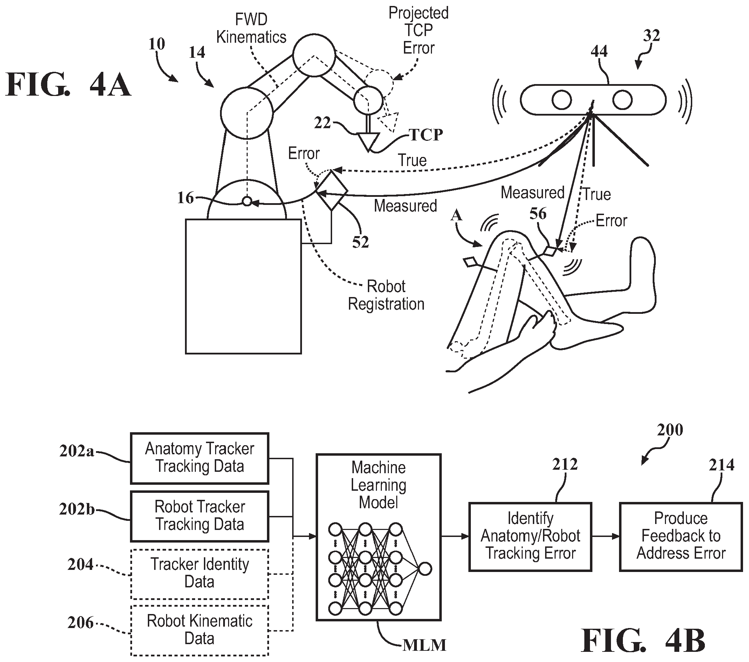

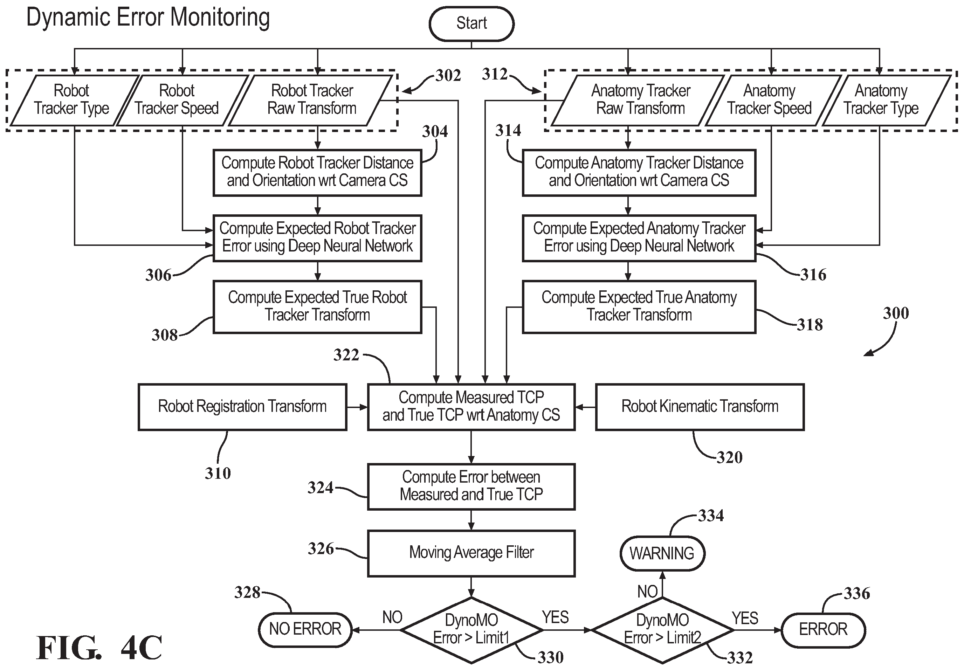

Having described the method 200 for tracking error identification and compensation, this section now described one example use case of this technique, namely, dynamic error monitoring, which is provided with reference to FIGS. 4A, 4B, 4C and methods 200, 300.

For dynamic error monitoring, a manipulator tracker 52 is coupled to a component of the robotic manipulator 14 and an anatomy tracker 56 is coupled to the anatomy (A). In the example shown, the manipulator tracker 52 is coupled to the base 16 of the manipulator 14 via an adjustable support arm and the anatomy tracker 56 is coupled to a patient's tibia. However, as described above, the manipulator tracker 52 can be coupled to any component of the manipulator 14, such as one of the links 18, the end effector 22, or a coupler between the robotic arm and the end effector 22. Similarly, the anatomy tracker 56 can be coupled to any respective anatomy depending on the nature of the procedure.

One aspect of dynamic error monitoring is to utilize the techniques described above to predict dynamic error due to anatomical and/or localizer 44 motion during a surgical procedure, such as robotic arthroplasty. The monitoring aspect can serve as a surveillance technique to protect patients from undesirable robotic actions caused by localizer 44 performance degradation due to dynamic anatomical motion resulting in a considerable orientation change in anatomy tracker 56. Orientational tracking error, when projected onto another coordinate system, can introduce a significant additional translation error on top of the existing error. By identifying the tracking error, the controller(s) can estimate or predict TCP inaccuracies or cutting error at the end effector 22. Similarly, movement of the localizer 44 can impact the accuracy of both the manipulator tracker 52 as well as the anatomy tracker 56.

To implement this dynamic error monitoring technique, as shown in FIG. 4B, the localizer 44 detects a pose of the manipulator tracker 52 and a pose of the anatomy tracker 56 and produces tracking data (202a, 202b) related to each tracker 52, 56, respectively, which can include pose and velocity/acceleration. Tracker identity data (204) of each tracker 52, 56 can be acquired, as described above. Additionally, surgical object data (206), in this case, kinematic data from the manipulator 14, can be acquired using joint encoder measurements. Using forward kinematic calculations, the tracking system 32 can determine a relationship between the base 16 of the manipulator 14 and the TCP of the end effector 22. Similarly, using tracking data, transforms can be computed between: (1) the base 16 of the manipulator and the manipulator tracker 52; (2) the manipulator tracker 52 and the localizer 44; (3) the localizer 44 and the anatomy tracker 56; and (4), the anatomy tracker 56 to the target anatomy (A). From these various transforms, relationships can be determined between the localizer 44 and the base 16 and/or between the TCP and the target anatomy (A).

The controller(s) obtain the real-time tracking data from the localizer 44, and optionally, the kinematic data and tracker identity data, and input the data into the machine learning model (MLM). The machine learning model (MLM) can utilize this information to define an interactive pre-state formed by the anatomy state and the end effector state. The machine learning model (MLM) is configured to identify the error (212) related to the detected pose of one or both of the manipulator tracker and the anatomy tracker 56. The identified error can be an actual/predicted/estimated cutting error at the TCP.

The machine learning model (MLM) intelligently and selectively evaluates whether the tracking error should be addressed or disregarded. To elaborate, conventional tracking systems typically compensate for tracking errors by halting system operation in response to the tracking error strictly exceeding a default error threshold and implement this scheme for any tracker and for any situation, without discrimination. Contrarily, the machine learning model (MLM) can provide a customized approach to handling the tracker error and can do so by considering several important factors, such as a type of tracker, the type or context of tracker movement, the source of the error, or a context of a surgical procedure. The machine learning model (MLM) can selectively evaluate whether the tracking error would be acceptable given the circumstances or context in which the error occurred. Accordingly, using this approach, the tracking system 32 would be less susceptible to flagging tracking errors thereby minimizing disruptions to the surgical procedure. To implement this technique, the machine learning model (MLM) can establish an error threshold/range/gauge that dynamically changes based on the input data (tracking data, identity data, surgical object data) and/or identified error. The error threshold/range/gauge can change for each instance of tracking data. The machine learning model (MLM) can identify the error in response to the error exceeding the dynamically changing error threshold/range/gauge.

To further illustrate, the machine learning model (MLM) can estimate the localization error for the given anatomy and robot end effector state by establishing a real-time risk assessment factor by projecting the localization error out to the tool center point (TCP) of the manipulator 14 which is measured in the anatomy coordinate system. For example, in FIG. 4A, measured and true (actual) tracker measurements are illustrated with respect to the localizer 44, wherein the measured tracker measurements are indicative of those that include tracking errors. In the illustrated example, the tracking errors occur at the anatomy tracker 56 and manipulator tracker 52 due to unexpected movement of the localizer 44. Using the transforms described, the tracking error impact can be projected to the TCP, and the risk assessment can be made based on this projected error. The machine learning model (MLM) can establish the real-time risk assessment factor by quantifying the dynamic error due to the anatomy motion or localizer 44 movement, e.g., during robotics-assisted arthroplasty. With these techniques, the machine learning model (MLM) can make various estimations about the impact of the error on the system. For example, the machine learning model (MLM) can: estimate the impact of dynamic anatomical motion on anatomy localization; estimate the impact of localizer movement on robot tracking performance; estimate the impact of localizer movement on anatomy localization; estimate the impact of robot arm motion/robot cart movement on robot tracking performance; or predict the dynamic error due to localizer movement, robot cart/arm motion, and dynamic anatomical motion.

The machine learning model (MLM) can selectively judge whether the real-time risk assessment factor is greater than the dynamic threshold/range/gauge. If the real-time risk assessment factor is greater than the dynamic threshold/range/gauge, the controller(s) produce feedback (214) to address the error, such as by producing a warning or notification using the techniques described above. Such warnings can be provided to avoid cutting error, reducing the operation risk, and protecting patients from undesirable cutting resulting from the localizer performance degradation due to tracker detection inaccuracies. The controller(s) can also present the expected TCP error in real time on any of the displays 38, 90. If the TCP error exceeds the dynamic threshold/range/gauge, the source of the error can also be presented, such as: localizer movement, anatomy movement, robot cart movement, or excessive robotic arm movement with high orientation variation. Such notifications or warnings can provide valuable insights into the precautionary steps the surgeon can take to minimize the impact of localization errors on TCP accuracy. Other forms of feedback are contemplated, such as those described above, such as providing a command to modify or halt operation of the robotic manipulator 14.

If the real-time risk assessment factor is less than the dynamic threshold/range/gauge, the controller(s) may selectively disregard the error. Consequently, the controller(s) may allow movement of the localizer 44 and/or target anatomy as long as the expected dynamic error does not exceed the accuracy requirement.

The method 300 of FIG. 4C provides another implementation of the dynamic error monitoring technique and this method can be further understood with reference to the setup and transforms illustrated in FIG. 4A. Starting with the manipulator (robot) tracker 52, the controller(s) acquire manipulator tracker data at step 302. This data can include tracking data from the localizer 44, including pose and velocity of the manipulator tracker 52, as well as tracker identity or configuration/type data, as described above. At 304, the controller(s) can utilize raw tracking data from the localizer 44 to compute a pose (or distance) and orientation of the manipulator tracker 52 with respect to a coordinate system (CS) of the localizer 44. At 306, utilizing the computed pose (or distance) and orientation of the manipulator tracker 52 (determined at 304), and optionally, the manipulator tracker 52 velocity and tracker identity/type data (from 302), the controller(s) then compute an expected error of the manipulator tracker 52. In one implementation, the controller(s) implements the machine learning model (MLM) to compute the expected error, using any of the techniques described above. The machine learning model (MLM) may be a deep neural network, or any other suitable type of model. At 308, the controller(s) utilizes the expected error of the manipulator tracker 52 to compute an expected true transform of the manipulator tracker 52. The expected true transform represents the location at which the manipulator tracker 52 would be expected to be positioned within the coordinate system of the localizer 44 if the error were not present.

With respect to the anatomy tracker 56, at 312, the controller(s) acquire anatomy tracker data. This data can include tracking data from the localizer 44, including pose and velocity of the anatomy tracker 56, as well as tracker identity or configuration/type data, as described above. At 314, the controller(s) utilize raw tracking data from the localizer 44 to compute a pose (or distance) and orientation of the anatomy tracker 56 with respect to a coordinate system (CS) of the localizer 44. At 316, utilizing the computed pose (or distance) and orientation of the anatomy tracker 56 (determined at 314), and optionally, the anatomy tracker 56 velocity and tracker identity/type data (from 312), the controller(s) then computes an expected error of the anatomy tracker 56. In one implementation, the controller(s) implements the machine learning model (MLM) to compute the expected error, using any of the techniques described above. At 318, the controller(s) utilize the expected error of the anatomy tracker 56 to compute an expected true transform of the anatomy tracker 56. The expected true transform represents the location at which the anatomy tracker 56 would be expected to be positioned within the coordinate system of the localizer 44 if the error were not present.

At 310, the controller(s) can obtain a robot registration transform, indicative of the relationship between the manipulator tracker 52 and the base of the manipulator 16. This can be acquired using any suitable robot registration process, which are described in detail below. At 312, the controller(s) can further obtain kinematic data from the manipulator 14 to determine a relationship between the TCP and the base 16. Using the computed expected true transforms (308, 318), tracking data (302, 312), the robot registration transform (310), and the kinematic transform (320), the controller(s) can compute a measured TCP value and true TCP value with respect to a coordinate system of the anatomy (at 322). Hence, the tracking error can be projected to the TCP of the robot. The controller(s) at 324 can then compute an error between the measured and true TCP values, which is indicative of the error resulting from dynamic motion of any one of the trackers 52, 56. A filtering or smoothing of the computed error can optionally be implemented at 326 to avoid over-reactive feedback. At 330, the controller(s) determine whether the computed error exceeds a first threshold. If not, the controller(s) conclude there is no tracking error at 328. If so, the controller(s) can compare the computed error to a second threshold at 332. If the computed error is below the second threshold, the controller(s), at 334, can provide feedback via a notification or warning to inform a user of the error, using any of the techniques described in this document. This feedback may indicate that there is a potential error, which has not yet reached the level of a threshold error, to provide the surgeon with an opportunity to take corrective actions before identification of the threshold error. If the computed error is above the second threshold, the controller(s), at 336, can take more aggressive actions, such as by providing a threshold error indicator and/or modifying control of the manipulator 14, such as halting the manipulator 14, or by providing any other feedback described above.

The dynamic error monitoring techniques include several advantages. These described techniques replace conventional, hard coded constraints, which are insufficient for complex error detection. The dynamic error monitoring techniques can generalize to a wider range of operating conditions and scenarios and are well suited to evaluate the inputs from multiple disparate data sources (e.g., localizer, anatomy, and manipulator rotation and translation) simultaneously and accurately. The solutions also provide the additional benefit of being tolerant of the noise generated by sensors and the environment.

### C. Robot Registration

This section describes another example of utilizing the error identification techniques described above, namely, for enhancing the robot registration process. Robot registration is a process that is performed before surgery is executed and involves aligning/registering a coordinate system of the manipulator 14 with a coordinate system of the localizer 44. Accurate registration of the manipulator 14 is necessary to ensure the manipulator 14 can be accurately tracked relative to the patient to perform the procedure with accuracy. However, robot registration is particularly sensitive to tracking errors due to the localizer's 44 susceptibility to orientation changes during data collection. Such errors may occur when the trackers involved in the registration process do not consistently face the localizer 44 or experience unexpected movements, including non-linear rotations.

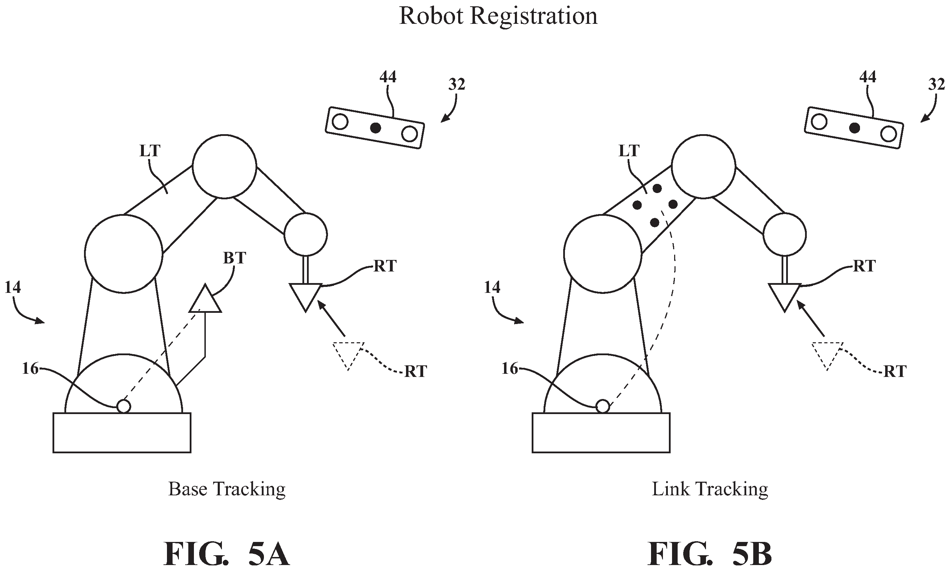

With reference to FIGS. 5A-5D, the robot registration process and result may differ from one system to the next depending on the specific configuration of the manipulator trackers located on the manipulator 14. As will be described below, these manipulator trackers can be located on various components of the manipulator 14, such as, but not limited to: the base 16, links 18, the end effector 22, etc.

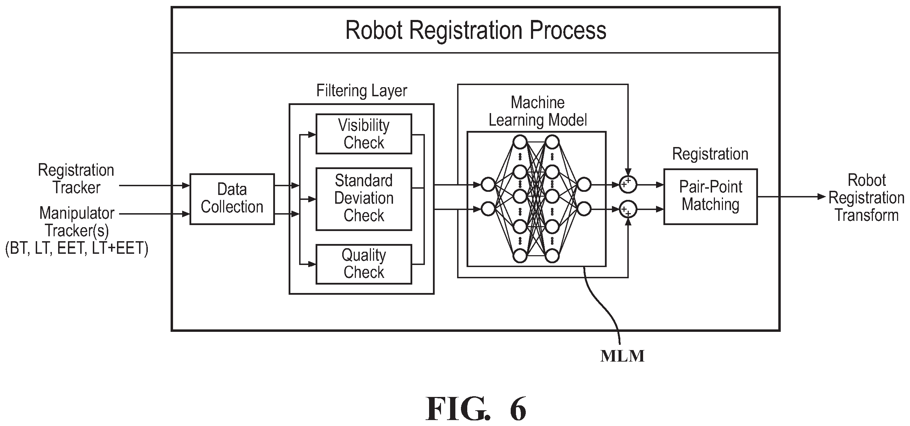

In FIG. 5A, the manipulator tracker is a base tracker (BT) located on an adjustable arm that is coupled to the base 16 of the manipulator 14. Since the arm is adjustable in various poses that can be set by a user, the relationship between the base tracker (BT) and the base 16 must be determined using the robot registration process. A user then attaches a registration tracker (RT) to the end effector 22 in a predetermined relationship. One example of the registration tracker (RT) can be like the end effector tracker (47) (see FIG. 6B) of US Patent No. 8,010,180, entitled "Haptic Guidance System And Method", the entire contents of which are hereby incorporated by reference. The registration tracker (RT) can include a coupling device, such as a clamp, which attaches to a tool extending from the end effector 22. The registration tracker (RT) may include a registration surface that abuts a TCP of the tool to define the predetermined relationship. The registration tracker (RT) is utilized only during the robot registration process and is removed thereafter. Once the registration tracker (RT) is attached, the system 10 commands the user to perform the robot registration process by moving the TCP in registration motion, such as by moving the TCP along edges and vertices of a cube haptic object that constrains the TCP to the vertices and edges. During this registration motion, localization data and kinematic data for the TCP are simultaneously acquired. Specifically, localization data is acquired from the registration tracker (RT) and kinematic data about the TCP is acquired from the forward kinematic calculations. These data points are paired and compared until an accuracy threshold is met.

The example of FIG. 5B involves use of a link tracker (LT) associated with one of the links 18 of the manipulator 14, instead of the base tracker (BT). The link tracker (LT) may be in a fixed relationship relative to the manipulator 14 or may be in a user-defined, or custom location relative to the manipulator 14. For example, the link tracker (LT) can be attached anywhere on one or more links 18, without prior knowledge of the reference transform to the nearest joint of the manipulator 14. During the robot registration process, this missing transformation is computed, which can later be used in conjunction with the kinematics of the manipulator 14 to determine the link tracker's (LT) position relative to the robot base 16. Even when the link tracker (LT) is in a fixed relationship to the manipulator 14, there are factors that may affect this relationship, such as mechanical compliance of the links or joints of the robotic arm. Accordingly, the robot registration process should still be performed to accurately determine the relationship between the link tracker (LT) and the base 16. Whether the link tracker (LT) is fixed or custom-defined, the robot registration process similarly includes attaching the registration tracker (RT) to the TCP, moving the manipulator 14 in a registration motion, and acquiring kinematic/localization data pairs until sufficient accuracy is achieved to define the relationship between the link tracker (LT) and the base 16. The relationship to the base 16 can be established using several intervening transforms, such as between the link tracker (LT) and the links 18 of the manipulator 14.

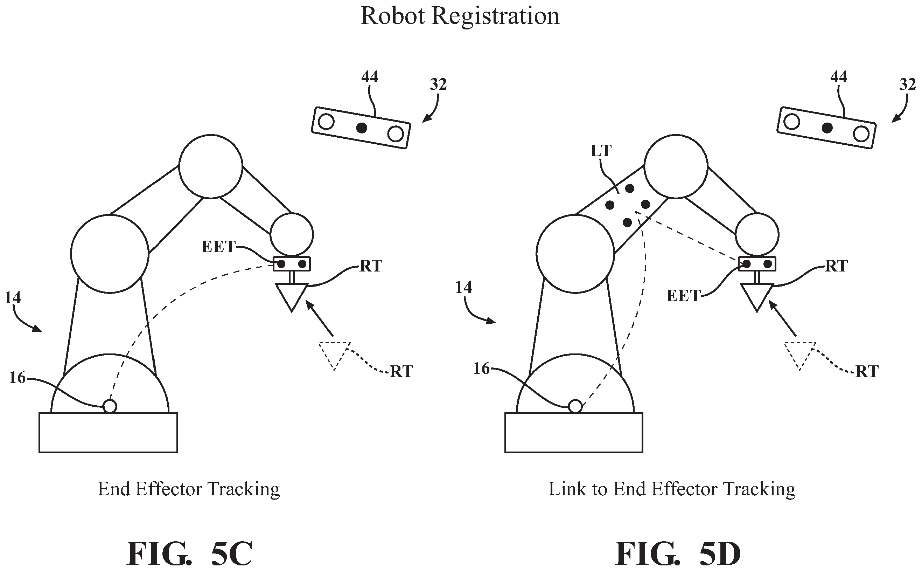

In the example of FIG. 5C, the manipulator 14 includes an end effector tracker (EET), i.e., without a base tracker or link tracker. The end effector tracker (EET) may be incorporated into the body of the end effector 22, attachable to the end effector 22, or may be provided on a coupler between the end effector 22 and the distal flange of the robotic arm. Such a coupler can be like that described in US Patent App. Pub. No. 20220039898, entitled, "Robotic Surgical System Including A Coupler For Connecting A Tool To A Manipulator And Methods Of Using The Coupler", the entire contents of each of these references being hereby incorporated by reference. The robot registration process similarly includes attaching the registration tracker (RT) to the TCP, moving the manipulator 14 in a registration motion, and acquiring kinematic/localization data pairs until sufficient accuracy is achieved to define the relationship between the end effector tracker (EET) and the base 16. The relationship to the base 16 can be established using several intervening transforms, such as between the end effector tracker (EET) and the distal flange of the manipulator 14.

The example of FIG. 5D is like that of FIG. 5C, with the addition of the link tracker (LT). Here, the end effector tracker (EET) and link tracker (LT) are used in combination. The robot registration process similarly includes attaching the registration tracker (RT) to the TCP, moving the manipulator 14 in a registration motion, and acquiring kinematic/localization data pairs until sufficient accuracy is achieved to define the relationship between: the link tracker (LT) and the base 16; the end effector tracker (EET) and the base 16; and/or the link tracker (LT) and the end effector tracker (EET) combined, relative to the base 16. The relationship to the base 16 can be established using several intervening transforms, such as between the link tracker (LT) and the links 18 of the manipulator 14 or between the end effector tracker (EET) and the distal flange of the manipulator 14.

Having described the various forms of manipulator tracking for robot registration, we now describe how the machine learning techniques described above can enhance the robot registration for any given variation thereof. The proposed compensation scheme during the robot registration process will significantly improve the accuracy of the robot registration transform, particularly when the manipulator tracker is dynamically moving. The robot registration transform obtained from the proposed compensation scheme improves robustness of the system in view of camera motion without impacting the cutting accuracy.

With reference to FIG. 6, the method includes obtaining tracking data from the registration tracker (RT) and the manipulator tracker(s), which are obtained by the localizer 44 during the robot registration process. Again, the manipulator tracker(s) can include a base tracker (BT), link tracker (LT), end effector tracker (EET), or any combinations thereof. Supplemental data regarding the tracker identities can be obtained. Information about the robotic kinematics is also acquired by the controller(s). The tracking data (and optionally supplemental data) is inputted into the machine learning model (MLM). The machine learning model (MLM) identifies an error related to the detected pose of one or both of the manipulator tracker and the registration tracker and can produce feedback to address the error.

In one example implementation, as shown in FIG. 6, the tracking data may optionally be provided into a coarse filtering layer before being provided as input to the machine learning model (MLM). The coarse filtering layer can provide filtering of the tracking data by disregarding tracking data that fails certain checks. One check is a visibility check, wherein the controller filters out tracking data that fails to include all visible markers from both the manipulator and the registration tracker (RT). A second check is a standard deviation check, which is applied in static data collection scenarios. Here, multiple data points are collected for a single pose, and the controller(s) calculate their standard deviation. If the standard deviation exceeds a predetermined threshold, indicating that the pose was not stationary, the data is discarded. The standard deviation check can be used to confirm that the localizer 44 and manipulator 14 have remained stationary. A third check is a quality check, wherein the controller(s) assesses the quality of the tracking data to ensure it meets a predefined threshold before being used in the registration process. Only data that surpasses this quality threshold is retained for further use.

The filtered tracking data is then input into the machine learning model (MLM) for enhancing the accuracy of robot registration. The machine learning model (MLM) in this example, serves to provide a more advanced filtering layer for the tracking data to reduce, eliminate or compensate for tracking errors, e.g., using any of the techniques described above. The machine learning model (MLM) can determine or predict errors that may be expected for the specific tracker configurations used in the robot registration process and compensate for the expected error before using it in the robot registration process. The machine learning model (MLM) can modify tracking data to reduce/eliminate expected error in several ways, such as replacing tracking data with more accurate tracking data, changing values of tracking data, or omitting inaccurate tracking data. The machine learning model (MLM) can filter the tracking data continuously or at discrete times. For example, the machine learning model (MLM) can filter the tracking data, in real-time, as it is received during the robot registration process. Alternatively, the machine learning model (MLM) may filter the tracking data after acquisition of all tracking data during the robot registration process.

To identify the robot registration transform, the tracking/kinematic data pair points are matched. In this case, the controller(s) can obtain more than one robot registration transform. One version of the transform can be based on pair-point matching based on data that has been filtered by the machine learning model. This transform may be considered a machine-learning based robot registration transform. A second version of the transform can be based on pair-point matching based on data output directly from the filtering layer (i.e., without any filtering from the machine learning model). This transform may be considered a standard robot registration transform. The controller(s) can present the results of the standard and machine-learning based robot registration transforms to the user on the display 38, 90, using any suitable format. The controller(s) can present statistics about, or comparisons between, the standard and machine-learning based registrations. For example, the display 38, 90 can provide accuracy statistics, such as an overall accuracy score of each registration process. Pursuant to providing such information, the controller(s) can prompt the user to select one of the transforms. If the user sees that the machine-learning based registration provided more accurate results, the user can select that result, for example.

In another example, a verification process can be performed after robot registration. Using the outcome of this verification process, the controller(s) can automatically determine which registration method (standard or ML-enabled) to use. In one example, the verification process can involve tracking the registration tracker (RT) as the manipulator 14 is moved to one or more predetermined positions (e.g., aiming end effector to the floor/ceiling). In another example, the pointer P can be used to touch a divot or checkpoint provided on a known location relative to the manipulator 14, such as on the surgical tool. In another example, both the localizer 44 and the manipulator 14can be moved, and statistical measures can be presented to the surgeon to aid in selecting the registration method that is more robust to localizer movement. The localizer movement can be performed either manually or autonomously, for example, if the localizer is mounted to a separate manipulator.

### D. Anatomical Registration

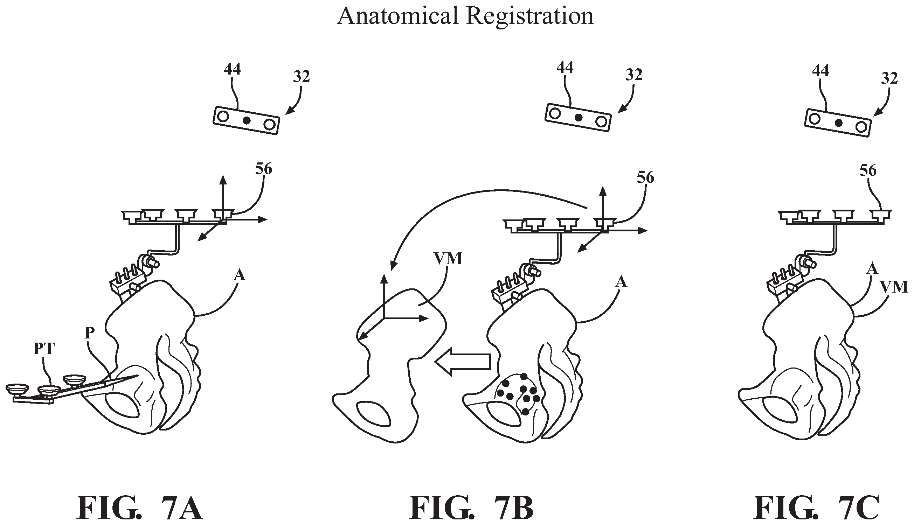

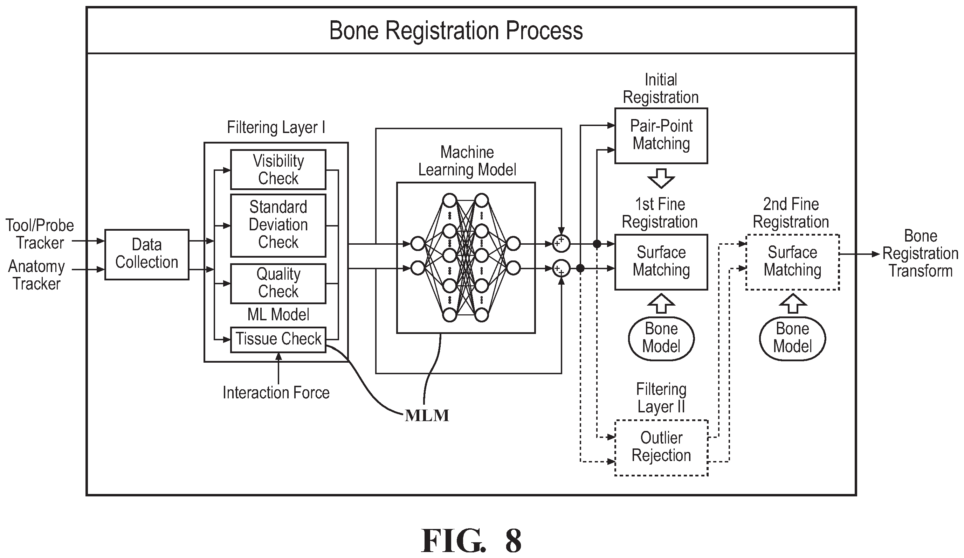

Yet another example of utilizing the error identification techniques is described in this section, namely, for enhancing the anatomical registration process. With reference to FIGS. 7 and 8, anatomical registration is a process that is performed before surgery is executed and involves aligning/registering a coordinate system of a virtual anatomical model (AM) (which includes aspects of a surgical plan) with the target anatomy (A) being tracked, and hence, with the coordinate system of the localizer 44. The anatomy (A) being tracked can be any anatomy, such as any bone, organ, or the like. In the example shown, the anatomy being tracked is a bone, specifically a pelvic bone, which is being tracked by an anatomy tracker 56. The virtual model (VM) includes surgical planning aspects, such as a planned position of an implant, planned regions/volumes of the anatomy to be cut, virtual constraint boundaries, and the like. The virtual model (VM) is derived from patient imaging data, such as CT data, which can be segmented to form the virtual model (VM). Accurate registration of the anatomy (A) is necessary to ensure the manipulator 14 can be accurately tracked relative to the patient to perform the procedure with accuracy. However, anatomical registration is susceptible to the tracking errors described above because the trackers involved with anatomical registration may not perfectly face the localizer 44 or may experience unexpected movements or a wide range of orientational changes, such as non-linear rotations.

With reference to FIGS. 7A-7C, one example of performing the anatomical registration process is provided using a pointer or probe tool (P).

In FIG. 7A, the anatomy tracker 56 is coupled directly to the anatomy A. Since the anatomy tracker 56 is manually attached by a user, the relationship between the anatomy tracker 56 and the anatomy (A) must be determined using the anatomical registration process. Accordingly, a user utilizes the pointer/probe tool (P), which is equipped with a tool tracker (PT), in this case a pointer tracker, which has a known and fixed relationship to the tip of the probe tool (P). The probe tool (P) tip position can be accurately determined in the coordinate system of the localizer 44. One example of the probe tool (P) and tool tracker (PT) can be like the probe and tracker (see FIG. 7) of US Patent No. 8,010,180, entitled "Haptic Guidance System And Method", the entire contents of which are hereby incorporated by reference. Once the tool tracker (PT) is detectable by the localizer 44, the system 10 commands the user, via the displays 38, 90, to perform the anatomical registration process by moving the tip of the probe tool (P) into contact with a target anatomical surface. The target surface of the anatomy is then digitized using the probe tool (P). In FIG. 7A, the target surface is an acetabulum of the pelvic bone. However, the target anatomical surface may vary depending on the procedure.

The probe tool (P) may be used in various workflows. For example, the tracking system 32 may require the user to touch the tip of the probe tool (P) to discrete identified landmark points. Additionally, or alternatively, a region-based registration technique may be utilized where the system may allow the user to touch various points within a specific region determined by the tracking system 32. In another example, the user may drag/sweep/trace the tip of the probe tool (P) across the target anatomical surface, thereby providing a continuous acquisition points or lines. There may be an initial (coarse) anatomical registration procedure followed by a second (fine) anatomical registration procedure. Such point/region and initial/fine registration processes can be like that described in US Patent No. US 10485450, entitled "Systems and methods for intra-operative pelvic registration", the entire contents of which are hereby incorporated by reference.

The tracking system 32 may include an input device, such as foot pedal or button on the probe tool (P) to command acquisition of any point(s) when the user determines the time for acquisition is appropriate. In other examples, the acquisition may be automatically triggered based on supplemental sensing. For example, when the probe tool (P) comprises a force sensor, or other tissue detecting sensor, measurements from the sensor(s) may indicate confidence that the tool tip is contacting the appropriate tissue (e.g., cortical bone) to trigger automatic acquisition of the point(s).

During this registration process, localization data from the tool tracker (PT) is acquired. Based on the relationship between the tool tracker (PT) and the tool tip, the position of the acquired points can be associated with the coordinate system of the localizer 44. Simultaneously, localization data from the anatomy tracker 56 is acquired and the acquired points are associated with the coordinate system of the anatomy tracker 56, and hence, the coordinate system of the localizer 44. The digitized points can then be mapped to the virtual model (VM), as shown in FIG. 7B. These tracking data points are paired and compared until an accuracy threshold is met. In turn, the virtual model (VM) can be registered to the physical anatomy (A), as shown in FIG. 7C.

The process of anatomical registration can differ from that shown depending on the specific techniques and tracking methods used during registration. The error compensation techniques described herein may be utilized with any other registration method. For example, instead of the probe tool (P), any other tracked instrument that includes a tool tip may be utilized to acquire points, such as a tool attached to the manipulator 14, a tool tip attached to a motorized or robotic hand-held tool (such as a drill, screwdriver, tap, awl, saw, router, cutting bur, etc.). Also, "imageless" anatomical registration can be utilized wherein there exists no virtual model (VM) of the anatomy. Instead, a virtual surface of the anatomy (A) is derived intraoperatively from a point cloud acquired using the probe tool (P) and the surgical plan can be intraoperatively developed relative to the virtual surface. In some imageless registration techniques, the anatomy may not necessarily be tracked. In other examples, anatomical registration may involve using computer/machine vision tracking to derive the surface of the anatomy, e.g., using a depth map, wherein the device that performs the computer/machine vision tracking may be tracked by the localizer 44. Such vision techniques can be implemented by the HMD or by the localizer 44 attached to the cart. In other implementations, the user's hand and finger(s) can be tracked by any vision tracking system and the user can touch the target anatomical surface with their fingers to acquire points. In another instance, an ultrasound imaging device that is tracked by the localizer 44 may be utilized to perform anatomical registration. Anatomical registration can be performed with any suitable tracking modality.

Having described the various forms of tracking for anatomical registration, we now describe how the machine learning techniques described above can enhance the anatomical registration for any given variation thereof. The proposed compensation scheme during anatomical registration process will significantly improve the accuracy of the anatomical registration transform, particularly when the tool tracker (PT) and/or anatomy tracker 56 are dynamically moving. The anatomical registration transform obtained from the proposed compensation scheme improves robustness of the system in view of camera motion without impacting the cutting accuracy.

With reference to FIG. 8, the method includes obtaining tracking data from the anatomy tracker 56 and tool tracker (PT), which are obtained by the localizer 44 during the anatomical registration process. Supplemental data regarding the tracker identities can be obtained. When the probe tool (P) is equipped with force or tissue sensors, supplemental data from such sensors can also be acquired. The tracking data (and optionally supplemental data) is inputted into the machine learning model (MLM). The machine learning model (MLM) identifies an error related to the detected pose of one or both of the anatomy tracker 56 and the tool tracker (PT) and can produce feedback to address the error.

In one example implementation, as shown in FIG. 8, the tracking data may optionally be provided into a coarse filtering layer before being provided as input to the machine learning model (MLM). The coarse filtering layer can provide filtering of the tracking data by disregarding tracking data that fails certain checks. One check is a visibility check, wherein the controller(s) filters out tracking data that fails to include all visible markers from both the anatomy tracker and the registration tracker (RT). A second check is a standard deviation check, which is applied in static data collection scenarios. Here, multiple data points are collected for a single pose, and the controller calculates their standard deviation. If the standard deviation exceeds a predetermined threshold, indicating that the pose was not stationary, the data is discarded. A third check is a quality check, wherein the controller(s) assesses the quality of the tracking data to ensure it meets a predefined threshold before being used in the anatomical registration process. Only data that surpasses this quality threshold is retained for further use.

Additionally, the machine learning model (ML), or another machine learning model, may be involved with the coarse filtering layer by implementing a tissue check that is based on the sensors of the probe tool (P) that obtain measurements about the contacted tissue. Specifically, the controller(s) can obtain sensed measurements during interactions of the tool (P) with the tissue and apply the sensed measurements to a machine learning model that is configured to characterize a type of the tissue or distinguish distinct types of tissue. If the tissue identified is a soft tissue or cartilage, such acquired points can be discarded or filtered by the model. Such techniques can be like those described in U.S Provisional Patent Appl. No. 63/749,842, filed January 27, 2025, entitled "Robotic Surgical Systems And Methods Employing Machine Learning Models To Characterize Tool Interactions", the entire contents of which are hereby incorporated by reference.

The filtered tracking data is then input into the machine learning model (MLM) for enhancing the accuracy of anatomical registration. The machine learning model (MLM) is this example, serves to provide a more advanced filtering layer for the tracking data to reduce, eliminate or compensate for tracking errors, e.g., using any of the techniques described above. The machine learning model (MLM) can determine or predict errors that may be expected for the specific tracker configurations used in the anatomical registration process and compensate for the expected error before using it in the anatomical registration process. The machine learning model (MLM) can modify tracking data to reduce/eliminate expected error in several ways, such as replacing tracking data with more accurate tracking data, changing values of tracking data, or omitting inaccurate tracking data. The machine learning model (MLM) can filter the tracking data continuously or at discrete times. For example, the machine learning model (MLM) can filter the tracking data, in real-time, as it is received during the anatomical registration process. Alternatively, the machine learning model (MLM) may filter the tracking data after acquisition of all tracking data during the anatomical registration process.

To identify the anatomical registration transform, the tracking data from the anatomy tracker 56 and tool tracker (PT) are matched to corresponding points on the virtual model (VM). In this case, the controller(s) can obtain more than one anatomical registration transform. One version of the transform can be based on pair-point matching based on data that has been filtered by the machine learning model (MLM). This transform may be considered a machine-learning based anatomical registration transform. A second version of the transform can be based on pair-point matching based on data output directly from the filtering layer (i.e., without any filtering from the machine learning model). This transform may be considered a standard anatomical registration transform. The pair-point matching can be performed during the initial registration process and fine registration process, as described above. Anatomy registration can occur in two stages: an initial pair-point matching stage, which is similar to the robot registration process, followed by a surface matching stage. During the surface matching phase, outliers may be present in the collected data. If the initial surface matching indicates the presence of such outlier, e.g., detected by high residual errors or inconsistent point correspondences checked visually, the process can be repeated after applying an outlier rejection method (Filtering Layer II). This iterative refinement helps ensure that only highquality data contributes to the anatomical registration, thereby improving the overall accuracy and robustness of the resulting transform.

The controller(s) can present the results of the standard and machine-learning based anatomical registration transforms to the user on the display 38, 90, using any suitable format. The controller(s) can present statistics about, or comparisons between, the standard and machine-learning based registrations. For example, the display 38, 90 can provide accuracy statistics, such as an overall accuracy score of each registration process. In one instance, the controller(s) can present on the display(s) 38, 90, the results of the anatomical registration process with a 3D graphical representation of the virtual model (VM) of the anatomy provided with a heat map on a surface of the virtual model. The heat map can be colored and can identify the anatomical surfaces or regions that are subject to registration. The heat map can be used to compare accuracy between the standard and machine-learning based registrations. This will allow the surgeon to visually assess and confirm the improvement effectuated by machine learning, before the final mapping, thereby ensuring a better accuracy. Pursuant to providing such information, the controller(s) can prompt the user to select one of the transforms. If the user sees that the machine-learning based registration provided more accurate results, the user can select that result, for example.

Additionally, a verification process can be performed after anatomical registration. Using the outcome of this verification process, the controller(s) can automatically determine which registration method (standard or ML-enabled) to use. In one example, the verification process can involve using the pointer (P) to touch a divot or checkpoint provided on a known location relative to the anatomy (A).

### E. Extended-Reality

The error identification techniques described above can also be used with the HMD for extended reality purposes in the surgical context. As described above, the HMD may include the tracker 98 for which the tracking error can be identified using the machine learning model (MLM). In another example, the HMD can support the localizer 44, which can be the video camera 94 and/or vision sensing system. The HMD localizer 44 can detect other trackers for which the tracking error can be identified using the machine learning model (MLM).

In another example, when the localizer 44 is provided on the HMD, the localizer 44 can detect a pose of any object in an operation room and produce tracking data about the object. The object need not have a tracker attached thereto. For example, the object may be the hand of the surgeon, the hand of another person, a human body, a surgical instrument, the target anatomy, the manipulator 14, another HMD, or the like. When the motion is a hand motion, the hand motion can be for any purpose, such as providing gesture control input into the HMD, handling or operating a surgical tool, performing finger-point anatomical registration, or the like. The controller(s) can input the tracking data into the machine learning model (MLM) which can identify the tracking error related to the detected pose of the object and provide feedback to address the error related to tracking, using any of the techniques described above.

Several embodiments have been described in the foregoing description. However, the embodiments discussed herein are not intended to be exhaustive or limit the invention to any particular form. The terminology, which has been utilized, is intended to be in the nature of words of description rather than of limitation. Many modifications and variations are possible in light of the above teachings and the invention may be practiced otherwise than as specifically described.

The following CLAUSES define example implementations of the disclosed subject matter, with various features and combinations described herein being applicable individually or in combination, unless expressly stated otherwise:

[Dynamic error monitoring] A1. A surgical system comprising: a robotic manipulator comprising a robotic arm that is configured to support and move a surgical tool relative to an anatomy; and a surgical tracking system comprising: a manipulator tracker coupled to a component of the robotic manipulator; an anatomy tracker coupled to the anatomy; a localizer that is configured to detect a pose of the manipulator tracker and a pose of the anatomy tracker and produce tracking data related to the manipulator tracker and the anatomy tracker; and a control system coupled to the localizer and being configured to: obtain the tracking data from the localizer; input the tracking data into a machine learning model that is configured to identify an error related to the detected pose of one or both of the manipulator tracker and the anatomy tracker; and produce feedback to address the error. A2. The surgical system of clause A1, wherein the machine learning model is configured to: establish an error threshold or range that dynamically changes based on the tracking data; and identify the error in response to the error exceeding the error threshold or range. A3. The surgical system of clause A2, wherein the machine learning model dynamically changes the error threshold or range by being configured to estimate an impact that the error has on accuracy of one or more of: the localizer, the robotic manipulator, or the anatomy. A4. The surgical system of any preceding clause, wherein the control system provides feedback by being configured to provide a notification or warning to inform a user of the error. A5. The surgical system of any preceding clause, wherein the control system provides feedback by being configured to provide a command to modify or halt operation of the robotic manipulator. A6. A method of operating the surgical system of any preceding clause. A7. A computer program product configured to execute instructions to perform of the surgical system, control system, or machine learning model of any preceding clause.

[Dynamic Error] B1. A surgical system comprising: a robotic manipulator comprising a robotic arm that is configured to support and move a surgical tool relative to an anatomy, wherein the surgical tool include a TCP; and a surgical tracking system comprising: a manipulator tracker coupled to a component of the robotic manipulator; an anatomy tracker coupled to the anatomy; a localizer that is configured to detect a pose of the manipulator tracker and a pose of the anatomy tracker and produce tracking data related to the manipulator tracker and the anatomy tracker; and a control system coupled to the localizer and being configured to: obtain the tracking data from the localizer; based on the tracking data, compute expected errors for the manipulator tracker and the anatomy tracker; based on the expected error, compute expected true transforms of the manipulator tracker and the expected anatomy tracker; based on the tracking data and the expected true transforms, compute a measured TCP value and true TCP value with respect to a coordinate system of the anatomy; compute an error between the measured and true TCP values; and determine whether the error exceeds a threshold. B2. The surgical system of any preceding clause, wherein the control system implements a machine learning model to compute the expected errors. B3. The surgical system of clause B2, wherein the machine learning model is a deep neural network. B4. The surgical system of any preceding clause, wherein the tracking data includes velocities of the manipulator tracker and anatomy tracker. B5. The surgical system of any preceding clause, wherein to compute the expected errors for the manipulator tracker and the anatomy tracker, the control system further utilizes a tracker type for each of the manipulator tracker and anatomy tracker. B6. The surgical system of clause B1, wherein to compute the error between the measured and true TCP values, the control system further utilizes a registration transform of the manipulator derived from a robot registration process, and a kinematic transform of the manipulator. B7. The surgical system of clause B1, wherein if the error exceeds the threshold, the control system produces feedback to address the error. B8. The surgical system of clause B7, wherein the control system provides feedback by being configured to provide a notification or warning to inform a user of the error. B9. A method of operating the surgical system of any preceding clause. B10. A computer program product configured to execute instructions to perform of the surgical system, control system, or machine learning model of any preceding clause.

[Robot Registration] C1. A surgical system comprising: a robotic manipulator comprising a base and a robotic arm coupled to the base, wherein the robotic arm supports an end effector; and a surgical tracking system comprising: a manipulator tracker coupled to the robotic manipulator; a registration tracker configured to be attached to the end effector; a localizer configured to detect a pose of the manipulator tracker and a pose of the registration tracker and produce tracking data related to the manipulator tracker and the registration tracker; and a control system coupled to the robotic manipulator and the localizer and being configured to: obtain the tracking data from the localizer; input the tracking data into a machine learning model that is configured to identify an error related to the detected pose of one or both of the manipulator tracker and the registration tracker; and produce feedback to address the error. C2. The surgical system of any preceding clause, wherein the localizer detects the pose of the manipulator tracker and the pose of the registration tracker during a robot registration process in which the end effector and the registration tracker are moved relative to the base of the robotic manipulator, and wherein the robot registration process is performed to determine a relationship between the manipulator tracker and the base of the robotic manipulator. C3. The surgical system of clause C2, wherein the control system provides feedback by filtering the tracking data, with the machine learning model, to reduce or eliminate the error. C4. The surgical system of clause C3, wherein the control system is further configured to: obtain kinematic data from the robotic manipulator during the robot registration process; compare corresponding data pairs between the filtered tracking data and the kinematic data; and utilize the comparison to determine the relationship between the manipulator tracker and the base of the robotic manipulator, wherein the relationship is a first relationship. C5. The surgical tracking system of clause C4, wherein the control system is configured to: preserve tracking data that is unfiltered by the machine learning model; compare corresponding data pairs between the preserved tracking data and the kinematic data; and utilize the comparison to determine a second relationship between the manipulator tracker and the base of the robotic manipulator. C6. The surgical tracking system of clause C6, comprising a display device, and wherein the control system is configured to: present, on the display device, results of the robot registration process in accordance with the first relationship and the second relationship; and prompt a user to select one of the results of the robot registration process. C7. The surgical tracking system of clause C6, wherein the control system presents the results of the robot registration process with a comparison of accuracy between the first relationship and the second relationship. C8. The surgical system of any preceding clause, wherein the manipulator tracker is located on one or more of: an adjustable arm coupled to the base; at least one link of the robotic arm; and a coupler disposed between the robotic arm and the end effector. C9. A method of operating the surgical system of any preceding clause. C10. A computer program product configured to execute instructions to perform of the surgical system, control system, or machine learning model of any preceding clause.

[Anatomy Registration] D1. A surgical tracking system comprising: a tool tracker coupled to a surgical probe that is configured to interact with an anatomy; an anatomy tracker coupled to the anatomy; a localizer configured to detect a pose of the tool tracker and a pose of the anatomy tracker and produce tracking data related to the tool tracker and the anatomy tracker; and a control system configured to: obtain the tracking data from the localizer; input the tracking data into a machine learning model that is configured to identify an error related to the detected pose of one or both of the tool tracker and the anatomy tracker; and produce feedback to address the error. D2. The surgical tracking system of any preceding clause, wherein the localizer detects the pose of the tool tracker and the pose of the anatomy tracker during an anatomical registration process in which the surgical probe interacts with the anatomy, and wherein the anatomical registration process is performed to determine a relationship between the anatomy tracker and the anatomy. D3. The surgical tracking system of clause D2, wherein the control system provides feedback by filtering the tracking data, with the machine learning model, to reduce or eliminate the error. D4. The surgical tracking system of clause D3, wherein the control system is further configured to: obtain a virtual model of the anatomy; compare corresponding data pairs between the filtered tracking data and the virtual model; and utilize the comparison to determine the relationship between the anatomy tracker and the anatomy, wherein the relationship is a first relationship. D5. The surgical tracking system of clause D4, wherein the control system is configured to: preserve tracking data that is unfiltered by the machine learning model; compare corresponding data pairs between the preserved tracking data and the virtual model; and utilize the comparison to determine a second relationship between the anatomy tracker and the anatomy. D6. The surgical tracking system of clause D5, comprising a display device, and wherein the control system is configured to: present, on the display device, results of the anatomical registration process in accordance with the first relationship and the second relationship; and prompt a user to select one of the results of the anatomical registration process. D7. The surgical tracking system of clause D6, wherein the control system presents the results of the anatomical registration process with a 3D graphical representation of the virtual model of the anatomy with a heat map provided on a surface of the virtual model, wherein the heat map compares accuracy between the first relationship and the second relationship. D8. The surgical tracking system of any preceding clause, wherein: the surgical probe comprises a force sensor to produce force data responsive to the surgical probe interacting with tissue of the anatomy; the control system is configured to further input the force data into the machine learning model; and the machine learning model is further configured to identify a second error indicative of the surgical probe interacting with unwanted tissue. D9. A method of operating the surgical system of any preceding clause. D10. A computer program product configured to execute instructions to perform of the surgical system, control system, or machine learning model of any preceding clause.

## Claims

1. A surgical tracking system (10) comprising:
a tracker (52A, 52B, 54, 56, 98, PT, EET, RT, LT, BT) coupled to a surgical object;
a localizer (44) that is configured to detect a pose of the tracker (52A, 52B, 54, 56, 98, PT, EET, RT, LT, BT) and produce tracking data; and
a control system (60) coupled to the localizer (44) and being configured to:
obtain the tracking data from the localizer (44);
input the tracking data into a machine learning model (MLM) that is configured to identify an error related to the detected pose of the tracker (52A, 52B, 54, 56, 98, PT, EET, RT, LT, BT); and
produce feedback to address the error.

2. The surgical tracking system (10) of claim 1, wherein:
the control system (60) is further configured to acquire identity data associated with the tracker (52A, 52B, 54, 56, 98, PT, EET, RT, LT, BT), and input the identity data into the machine learning model (MLM) to enable the machine learning model (MLM) to identify the error;
and optionally,
the tracker (52A, 52B, 54, 56, 98, PT, EET, RT, LT, BT) comprises tracking elements (58) that are detectable by the localizer (44); and
the identity data comprises a geometry of the tracking elements (58).

3. The surgical tracking system (10) of any preceding claim, wherein:
the tracking data comprises a position of the tracker (52A, 52B, 54, 56, 98, PT, EET, RT, LT, BT) and an orientation of the tracker (52A, 52B, 54, 56, 98, PT, EET, RT, LT, BT);
and optionally,
the tracking data further comprises one or both of: a velocity of the tracker (52A, 52B, 54, 56, 98, PT, EET, RT, LT, BT) and an acceleration of the tracker (52A, 52B, 54, 56, 98, PT, EET, RT, LT, BT).

4. The surgical tracking system (10) of any preceding claim, wherein:
the machine learning model (MLM) comprises a neural network (NN);
and optionally,
the neural network (NN) comprises an input layer (IL), hidden layers (HL), and an output layer (OL), wherein the input layer (IL) is configured to receive the tracking data, and the output layer (OL) is configured to identify the error.

5. The surgical tracking system (10) of claim 5, wherein the machine learning model (MLM) comprises at least one long short-term memory (LSTM) coupled to the input layer (IL) of the neural network (NN), wherein the at least one LSTM comprises a recurrent neural network (RNN) and is configured to:
receive the tracking data over time;
modify weights of the RNN to learn long-term dependencies among the tracking data and to selectively filter the tracking data; and
provide filtered tracking data to the input layer (IL) of the neural network (NN).

6. The surgical tracking system (10) of any preceding claim, wherein the error relates to inaccuracy of the detected pose of the tracker (52A, 52B, 54, 56, 98, PT, EET, RT, LT, BT).

7. The surgical tracking system (10) of any preceding claim, wherein:
the machine learning model (MLM) is pre-trained based on ground-truth data derived from laser tracking of the tracker (52A, 52B, 54, 56, 98, PT, EET, RT, LT, BT) or other trackers; and
the machine learning model (MLM) identifies the error based on a comparison between the tracking data and the ground-truth data.

8. The surgical tracking system (10) of any preceding claim, wherein the machine learning model (MLM) is configured to:
establish an error threshold or range that dynamically changes based on the tracking data; and
identify the error in response to the error exceeding the error threshold or range.

9. The surgical tracking system (10) of any preceding claim, wherein the machine learning model (MLM) is configured to estimate an impact of the error on accuracy of tracking the surgical object.

10. The surgical tracking system (10) of any preceding claim, wherein:
the machine learning model (MLM) is further configured to predict a source of the error;
and optionally,
the source of the error comprises one or more of:
undesired movement of the surgical object;
undesired movement of the localizer (44); or
improper calibration data of the localizer (44) or the tracker (52A, 52B, 54, 56, 98, PT, EET, RT, LT, BT).

11. The surgical tracking system (10) of any preceding claim, wherein the control system (60) provides feedback by being configured to:
provide a notification or warning to inform a user of the error; and/or
selectively adjust or replace the tracking data to offset the error.

12. The surgical tracking system (10) of any preceding claim, wherein:
the tracker (52A, 52B, 54, 56, 98, PT, EET, RT, LT, BT) is an anatomy tracker (54, 56) coupled to an anatomy (A);
a manipulator tracker (52A, 52B, EET, RT, LT, BT) is coupled to a robotic manipulator (14) that includes a robotic arm that is configured to support and move a surgical tool (20) relative to the anatomy (A);
the localizer (44) is configured to detect a pose of the anatomy tracker (54, 56) and a pose of the manipulator tracker (52A, 52B, EET, RT, LT, BT) and produce the tracking data related to the anatomy tracker (54, 56) and the manipulator tracker (52A, 52B, EET, RT, LT, BT); and
the control system (60) is configured to:
obtain the tracking data from the localizer (44);
input the tracking data into the machine learning model (MLM) to identify the error related to the detected pose of one or both of the anatomy tracker (54, 56) and the manipulator tracker (52A, 52B, EET, RT, LT, BT); and
produce feedback to address the error.

13. The surgical tracking system (10) of any one of claims 1-11, wherein:
the tracker (52A, 52B, 54, 56, 98, PT, EET, LT, BT) is a manipulator tracker (52A, 52B, EET, RT, LT, BT) coupled to a robotic manipulator (14); and
a registration tracker (RT) is configured to be coupled to an end effector that is attached to a robotic arm supported by the base of the robotic manipulator (14);
the localizer (44) is configured to detect a pose of the manipulator tracker (52A, 52B, EET, RT, LT, BT) and a pose of the registration tracker (RT) and produce the tracking data related to the manipulator tracker (52A, 52B, EET, RT, LT, BT) and the registration tracker (RT); and
the control system (60) is configured to:
obtain the tracking data from the localizer (44);
input the tracking data into the machine learning model (MLM) to identify the error related to the detected pose of one or both of the manipulator tracker (52A, 52B, EET, RT, LT, BT) and the registration tracker (RT); and
produce feedback to address the error.

14. The surgical tracking system (10) of any one of claims 1-11, wherein:
the tracker (52A, 52B, 54, 56, 98, PT, EET, RT, LT, BT) is a tool tracker (PT) coupled to a surgical probe that is configured to interact with an anatomy (A);
an anatomy tracker (54, 56) is coupled to the anatomy (A);
the localizer (44) is configured to detect a pose of the tool tracker (PT) and a pose of the anatomy tracker (54, 56) and produce the tracking data related to the tool tracker (PT) and the anatomy tracker (54, 56); and
the control system (60) is configured to:
obtain the tracking data from the localizer (44);
input the tracking data into the machine learning model (MLM) to identify the error related to the detected pose of one or both of the tool tracker (PT) and the anatomy tracker (54, 56); and
produce feedback to address the error.

15. A computer-implemented method of operating a surgical tracking system (10), the surgical tracking system (10) including a tracker (52A, 52B, 54, 56, 98, PT, EET, RT, LT, BT) coupled to a surgical object, a localizer (44) that is configured to detect a pose of the tracker (52A, 52B, 54, 56, 98, PT, EET, RT, LT, BT) and produce tracking data, and at least one processor coupled to the localizer (44), the computer-implemented method comprising:
obtaining, with the at least one processor, the tracking data from the localizer (44);
inputting, with the at least one processor, the tracking data into a machine learning model (MLM) for identifying an error related to the detected pose of the tracker (52A, 52B, 54, 56, 98, PT, EET, RT, LT, BT); and
producing, with the at least one processor, feedback to address the error.
